# EUROPEAN PATENT APPLICATION

(11) **EP 4 122 956 A1**
(43) Date of publication of application: **25.01.2023**
(21) Application number: 21770767.8
(22) Date of filing: 19.03.2021
(51) Int. Cl.: C07K 14/725, C12N 15/12, C12N 15/867, C12N 5/10, A61P 35/00, A61K 39/00, A61K 48/00

(54) **HIGH-AFFINITY TCR FOR RECOGNIZING AFP ANTIGEN**

(30) Priority: 20.03.2020 CN 202010203369
(71) Applicant: XLifeSc, Ltd., Zhuhai, Guangdong 519031 (CN)
(72) Inventor: LI, Yi, Zhuhai, Guangdong 519031 (CN); PENG, Zhen, Zhuhai, Guangdong 519031 (CN); ZHENG, Hongjun, Zhuhai, Guangdong 519031 (CN); WU, Wanli, Zhuhai, Guangdong 519031 (CN)
(74) Representative: Grünecker Patent- und Rechtsanwälte PartG mbB
(86) International application number: PCT/CN2021/081897
(87) International publication number: WO 2021/185368

(57) **Abstract**

Provided is a T-cell receptor (TCR) that can bind to a KWVESIFLIF-HLA A2402 complex, wherein the binding affinity thereof is at least 2 times that of a wild-type TCR. Further provided is a fusion molecule of the TCR with a therapeutic agent, which fusion molecule targets a tumor cell presenting the KWVESIFLIF-HLA A2402 complex.

## Description

### TECHNICAL FIELD

The present disclosure relates to the field of biotechnology and, in particular, to a T-cell receptor (TCR) capable of recognizing a polypeptide derived from alpha-fetoprotein (AFP) protein. The present disclosure further relates to the preparation and uses of such receptors.

### BACKGROUND

There are only two types of molecules that can recognize antigens in a specific manner. One is immunoglobulin or antibody and the other is T-cell receptor (TCR), which is α/β or γ/δ heterodimeric glycoprotein on cell membrane. The TCR repertoire of an immune system is generated in thymus through V(D)J recombination, followed by positive and negative selections. In the peripheral environment, TCRs mediate the recognition of specific major histocompatibility complex-peptide complexes (pMHC) by T cells and, as such, are essential to the immunological functioning of cells in the immune system.

TCR is the only receptor for presenting specific peptide antigens in a major histocompatibility complex (MHC). The exogenous peptide or endogenous peptide may be the only sign of abnormality in a cell. In the immune system, once antigen-specific TCRs bind with pMHC complexes, it causes direct physical contact of a T cell and an antigen-presenting cell (APC). Then, the interaction of other membrane molecules in the T cell and APC occurs and the subsequent cell signaling and other physiological responses are initiated so that a range of different antigen-specific T cells exert immune effects on their target cells.

Molecular ligands of MHC class I and class II corresponding to TCRs are also proteins of the immunoglobulin superfamily but are specific for antigen presentation, and different individuals have different MHCs, thereby presenting different short peptides in one protein antigen to the surface of respective APC cells. Human MHC is commonly referred to as HLA gene or HLA complex.

Alpha-fetoprotein (AFP), also referred to as a-fetoprotein, is a protein expressed during embryonic development and is the main component of embryonic serum. During development, AFP is expressed at a relatively high level in the yolk sac and liver and then inhibited. In liver cancer, the expression of AFP is activated (Butterfield et al. J Immunol., 2001, Apr 15; 166(8): 5300-8). After generated in cells, AFP is degraded into micromolecular polypeptides and bind to MHC molecules to form complexes which are presented to cell surface. FMNKFIYEI is a short peptide derived from an AFP antigen and is a target for the treatment of AFP-related diseases.

Therefore, KWVESIFLIF-HLAA2402 complex provides a marker for TCR targeting tumor cells. The TCR capable of binding to KWVESIFLIF-HLA A2402 complex has high application value for treating tumors. For example, a TCR capable of targeting the tumor cell marker can be used to deliver a cytotoxic agent or an immunostimulatory agent to target cells, or have the same transformed into T cells such that the T cell expressing the TCR can destroy the tumor cells and can be administered to a patient during the course of treatment of so called adoptive immunotherapy. For the former purpose, the ideal TCR has higher affinity, allowing the TCR to reside on the targeted cells for a long period of time. For the latter purpose, it is preferred to use a TCR with medium affinity. Accordingly, those skilled in the art are directed to developing TCRs that can be used to target tumor cell markers for different purposes.

### SUMMARY

An object of the present disclosure is to provide a TCR with high affinity for KWVESIFLIF-HLA A2402 complex.

Another object of the present disclosure is to provide a preparation method for preparing the TCR of the preceding type and a use of the TCR of the preceding type.

In a first aspect of the present disclosure, a T-cell receptor (TCR) is provided, which comprises a TCRα chain variable domain and a TCRβ chain variable domain, and has an activity of binding to KWVESIFLIF-HLA A2402 complex. The TCRα chain variable domain comprises an amino acid sequence having at least 90% of sequence homology with an amino acid sequence as shown in SEQ ID NO: 1, and the TCRβ chain variable domain comprises an amino acid sequence having at least 90% of sequence homology with an amino acid sequence as shown in SEQ ID NO: 2.

In another preferred embodiment, the affinity of the TCR for KWVESIFLIF-HLA A2402 complex is at least twice that of a wild type TCR.

In another preferred embodiment, the wild type TCR comprises an α chain variable domain with an amino acid sequence as shown in SEQ ID NO: 1 and a β chain variable domain with an amino acid sequence as shown in SEQ ID NO: 2.

In another preferred embodiment, the TCRα chain variable domain comprises an amino acid sequence having at least 95% of sequence homology with the amino acid sequence as shown in SEQ ID NO: 1.

In another preferred embodiment, three complementarity-determining regions (CDRs) of the TCRα chain variable domain have the following reference sequences:
CDR1α: TSGFNG
CDR2α: NVLDGL
CDR3α: AVRHFSDGQKLL, and CDR3α contains at least one of the following mutations:

| Residue Before Mutation | Residue After Mutation |
|---|---|
| H at position 4 of CDR3α | G |
| F at position 5 of CDR3α | W |
| G at position 6 of CDR3α | Y or Q or K or D or A or H or I or L or M or N or R or T |
| D at position 7 of CDR3α | R or G or N |

In another preferred embodiment, three CDRs of the TCRα chain variable domain have the following reference sequences:
CDR1α: TSGFNG
CDR2α: NVLDGL
CDR3α: AVRHFSDGQKLL, and CDR3α contains the following amino acid mutation:

| Residue Before Mutation | Residue After Mutation |
|---|---|
| H at position 4 of CDR3α | G |

In another preferred embodiment, three CDRs of the TCRα chain variable domain have the following reference sequences:
CDR1α: TSGFNG
CDR2α: NVLDGL
CDR3α: AVRHFSDGQKLL, and CDR3α contains the following amino acid mutation:

| Residue Before Mutation | Residue After Mutation |
|---|---|
| F at position 5 of CDR3α | W |

In another preferred embodiment, three CDRs of the TCRα chain variable domain have the following reference sequences:
CDR1α: TSGFNG
CDR2α: NVLDGL
CDR3α: AVRHFSDGQKLL, and CDR3α contains the following amino acid mutations:

| Residue Before Mutation | Residue After Mutation |
|---|---|
| H at position 4 of CDR3α | G |
| F at position 5 of CDR3α | W |

In another preferred embodiment, three CDRs of the TCRα chain variable domain are:
CDR1α: TSGFNG;
CDR2α: NVLDGL; and
CDR3α: AVRHFSDGQKLL.

In another preferred embodiment, the TCRα chain variable domain has the amino acid sequence as shown in SEQ ID NO: 1.

In another preferred embodiment, the TCRβ chain variable domain comprises an amino acid sequence having at least 95% of sequence homology with the amino acid sequence as shown in SEQ ID NO: 2.

In another preferred embodiment, three CDRs of the TCRβ chain variable domain have the following reference sequences:
CDR1β: SGHRS
CDR2β: YFSETQ
CDR3β: ASSLGQGGKDEQY, and CDR3β contains at least one of the following mutations:

| Residue Before Mutation | Residue After Mutation |
|---|---|
| S at position 3 of CDR3β | A |
| L at position 4 of CDR3β | S |
| Q at position 6 of CDR3β | V |
| K at position 9 of CDR3β | R |
| D at position 10 of CDR3β | M or G |
| E at position 11 of CDR3β | Q or T |
| Q at position 12 of CDR3β | E or L |

In another preferred embodiment, three CDRs of the TCRβ chain variable domain are:
CDR1β: SGHRS;
CDR2β: YFSETQ; and
CDR3β: ASSLGQGGKDEQY

In another preferred embodiment, the TCRβ chain variable domain has the amino acid sequence as shown in SEQ ID NO: 2.

In another preferred embodiment, the TCRα chain variable domain comprises an amino acid sequence having at least 97% of sequence homology with the amino acid sequence as shown in SEQ ID NO: 1, and three CDRs of the TCRβ chain variable domain are:
CDR1β: SGHRS;
CDR2β: YFSETQ; and
CDR3β: ASSLGQGGKDEQY

In another preferred embodiment, the TCRα chain variable domain comprises an amino acid sequence having at least 97% of sequence homology with the amino acid sequence as shown in SEQ ID NO: 1, and the TCRβ chain variable domain comprises the amino acid sequence as shown in SEQ ID NO: 2.

In another preferred embodiment, the TCRβ chain variable domain comprises an amino acid sequence having at least 96% of sequence homology with the amino acid sequence as shown in SEQ ID NO: 2.

In another preferred embodiment, the TCRα chain variable domain comprises an amino acid sequence having at least 97% of sequence homology with the amino acid sequence as shown in SEQ ID NO: 1.

In another preferred embodiment, CDR3α of the TCR is selected from AVRHFSDGQKLL, AVRGWSDGQKLL, AVRGWYDGQKLL and AVRGWQDGQKLL.

In another preferred embodiment, CDR3β of the TCR is selected from AS SLGQGGKDEQY and ASSLGQGGRMQEY

In another preferred embodiment, three CDRs of the TCRα chain variable domain have the following reference sequences: CDR1α: TSGFNG; CDR2α: NVLDGL; and CDR3α: AVRHFSDGQKLL, wherein CDR3α contains at least one of the following mutations:

| Residue Before Mutation | Residue After Mutation |
|---|---|
| H at position 4 of CDR3α | G |
| F at position 5 of CDR3α | W |
| G at position 6 of CDR3α | Y or Q or K or D or A or H or I or L or M or N or R or T |
| D at position 7 of CDR3α | R or G or N |

and three CDRs of the TCRβ chain variable domain are CDR1β: SGHRS; CDR2β: YFSETQ; and CDR3β: ASSLGQGGKDEQY

In another preferred embodiment, three CDRs of the TCRα chain variable domain have the following reference sequences: CDR1α: TSGFNG; CDR2α: NVLDGL; and CDR3α: AVRHFSDGQKLL, wherein CDR3α contains at least one of the following mutations:

| Residue Before Mutation | Residue After Mutation |
|---|---|
| H at position 4 of CDR3α | G |
| F at position 5 of CDR3α | W |
| G at position 6 of CDR3α | Y or Q or K or D or A or H or I or L or M or N or R or T |
| D at position 7 of CDR3α | R or G or N |

and the TCRβ chain variable domain comprises the amino acid sequence as shown in SEQ ID NO: 2.

In another preferred embodiment, three CDRs of the TCRα chain variable domain have the following reference sequences: CDR1α: TSGFNG; CDR2α: NVLDGL; and CDR3α: AVRHFSDGQKLL, wherein CDR3α comprises the following amino acid mutations:

| Residue Before Mutation | Residue After Mutation |
|---|---|
| H at position 4 of CDR3α | G |
| F at position 5 of CDR3α | W |

and three CDRs of the TCRβ chain variable domain are CDR1β: SGHRS; CDR2β: YFSETQ; and CDR3β: ASSLGQGGKDEQY.

In another preferred embodiment, three CDRs of the TCRα chain variable domain have the following reference sequences: CDR1α: TSGFNG; CDR2α: NVLDGL; and CDR3α: AVRHFSDGQKLL, wherein CDR3α comprises the following amino acid mutations:

| Residue Before Mutation | Residue After Mutation |
|---|---|
| H at position 4 of CDR3α | G |
| F at position 5 of CDR3α | W |

and the TCRβ chain variable domain comprises the amino acid sequence as shown in SEQ ID NO: 2.

In another preferred embodiment, three CDRs of the TCRα chain variable domain are CDR1α: TSGFNG; CDR2α: NVLDGL; and CDR3α: AVRHFSDGQKLL, and the TCRβ chain variable domain comprises an amino acid sequence having at least 96% of sequence homology with the amino acid sequence as shown in SEQ ID NO: 2.

In another preferred embodiment, three CDRs of the TCRα chain variable domain are CDR1α: TSGFNG; CDR2α: NVLDGL; and CDR3α: AVRHFSDGQKLL, and three CDRs of the TCRβ chain variable domain have the following reference sequences: CDR1β: SGHRS; CDR2β: YFSETQ; and CDR3β: ASSLGQGGKDEQY, wherein CDR3β contains at least one of the following mutations:

| Residue Before Mutation | Residue After Mutation |
|---|---|
| S at position 3 of CDR3β | A |
| L at position 4 of CDR3β | S |
| Q at position 6 of CDR3β | V |
| K at position 9 of CDR3β | R |
| D at position 10 of CDR3β | M or G |
| E at position 11 of CDR3β | Q or T |
| Q at position 12 of CDR3β | E or L |

In another preferred embodiment, the TCRα chain variable domain comprises the amino acid sequence as shown in SEQ ID NO: 1, and three CDRs of the TCRβ chain variable domain have the following reference sequences: CDR1β: SGHRS; CDR2β: YFSETQ; and CDR3β: ASSLGQGGKDEQY, wherein CDR3β contains at least one of the following mutations:

| Residue Before Mutation | Residue After Mutation |
|---|---|
| S at position 3 of CDR3β | A |
| L at position 4 of CDR3β | S |
| Q at position 6 of CDR3β | V |
| K at position 9 of CDR3β | R |
| D at position 10 of CDR3β | M or G |
| E at position 11 of CDR3β | Q or T |
| Q at position 12 of CDR3β | E or L |

In another preferred embodiment, the TCRα chain variable domain comprises the amino acid sequence as shown in SEQ ID NO: 1, and the TCRβ chain variable domain comprises an amino acid sequence having at least 96% of sequence homology with the amino acid sequence as shown in SEQ ID NO: 2.

In another preferred embodiment, the TCRα chain variable domain comprises CDR1α, CDR2α and CDR3α, wherein the amino acid sequence of CDR1α is TSGFNG, the amino acid sequence of CDR2α is NVLDGL, and the amino acid sequence of CDR3α is AVR[3αX1][3αX2][3αX3][3αX4]GQKLL, wherein [3αX1] is H or G, [3αX2] is F or W, [3αX3] is G or Y or Q or K or D or A or H or I or L or M or N or R or T, and [3αX4] is D or R or G or N.

In another preferred embodiment, the TCRα chain variable domain comprises CDR1α, CDR2α and CDR3α, wherein the amino acid sequence of CDR1α is TSGFNG, the amino acid sequence of CDR2α is NVLDGL, and the amino acid sequence of CDR3α is AVR[3αX1][3αX2][3αX3][3αX4]GQKLL, wherein [3αX1] is H or G.

In another preferred embodiment, the TCRα chain variable domain comprises CDR1α, CDR2α and CDR3α, wherein the amino acid sequence of CDR1α is TSGFNG, the amino acid sequence of CDR2α is NVLDGL, and the amino acid sequence of CDR3α is AVR[3αX1][3αX2][3αX3][3αX4]GQKLL, wherein [3αX2] is F or W.

In another preferred embodiment, the TCRα chain variable domain comprises CDR1α, CDR2α and CDR3α, wherein the amino acid sequence of CDR1α is TSGFNG, the amino acid sequence of CDR2α is NVLDGL, and the amino acid sequence of CDR3α is AVR[3αX1][3αX2][3αX3][3αX4]GQKLL, wherein [3αX3] is G or Y or Q or K or D or A or H or I or L or M or N or R or T.

In another preferred embodiment, the TCRα chain variable domain comprises CDR1α, CDR2α and CDR3α, wherein the amino acid sequence of CDR1α is TSGFNG, the amino acid sequence of CDR2α is NVLDGL, and the amino acid sequence of CDR3α is AVR[3αX1][3αX2][3αX3][3αX4]GQKLL, wherein [3αX4] is D or R or G or N.

In another preferred embodiment, the TCR has a mutation in the α chain variable domain as shown in SEQ ID NO: 1, and the mutation is selected from one or more of H92G, F93W, G94Y/Q/K/D/A/H/I/L/M/N/R/T and D95R/G/N, wherein amino acid residues are numbered as shown in SEQ ID NO: 1.

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is AS[3βX1][3βX2]G[3βX3]GG[3βX4][3βX5][3βX6][3βX7]Y, wherein [3βX1] is S or A, [3βX2] is S or L, [3βX3] is Q or V, [3βX4] is K or R, [3βX5] is D or M or G, [3βX6] is E or Q or T, and [3βX7] is Q or E or L.

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is AS[3βX1][3βX2]G[3βX3]GG[3βX4][3βX5][3βX6][3βX7]Y, wherein [3βX1] is S or A.

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is A8[3βX1][3βX2]0[3βX3]00[3βX4][3βX5][3βX6][3βX7]Y, wherein [3βX2] is S or L.

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is A8[3βX1][3βX2]0[3βX3]00[3βX4][3βX5][3βX6][3βX7]Y, wherein [3βX3] is Q or V

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is A8[3βX1][3βX2]0[3βX3]00[3βX4][3βX5][3βX6][3βX7]Y, wherein [3βX4] is K or R.

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is A8[3βX1][3βX2]0[3βX3]00[3βX4][3βX5][3βX6][3βX7]Y, wherein [3βX5] is D or M or G.

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is AS[3βX1][3βX2]G[3βX3]GG[3βX4][3βX5][3βX6][3βX7]Y, wherein [3βX6] is E or Q or T.

In another preferred embodiment, the TCRβ chain variable domain comprises CDR1β, CDR2β and CDR3β, wherein the amino acid sequence of CDR1β is SGHRS, the amino acid sequence of CDR2β is YFSETQ, and the amino acid sequence of CDR3β is A8[3βX1][3βX2]0[3βX3]00[3βX4][3βX5][3βX6][3βX7]Y, wherein [3βX7] is Q or E or L.

In another preferred embodiment, the TCR has a mutation in the β chain variable domain as shown in SEQ ID NO: 2, and the mutation is selected from one or more of S94A, L95S, Q97V, K100R, D101M/G, E102Q/T and Q103E/L, wherein amino acid residues are numbered as shown in SEQ ID NO: 2.

In another preferred embodiment, the TCR has CDRs selected from the group consisting of:

| CDR No. | α-CDR1 | α-CDR2 | α-CDR3 | β-CDR1 | β-CDR2 | β-CDR3 |
|---|---|---|---|---|---|---|
| 1 | TSGFN G | NVLDG L | AVRGWSDGQ KLL | SGHRS | YFSET Q | ASSLGQGGK DEQY |
| 2 | TSGFN G | NVLDG L | AVRGWYDGQ KLL | SGHRS | YFSET Q | ASSLGQGGK DEQY |
| 3 | TSGFN G | NVLDG L | AVRHFSDGQ KLL | SGHRS | YFSET Q | ASSLGQGGR MQEY |
| 4 | TSGFN G | NVLDG L | AVRGWQDGQ KLL | SGHRS | YFSET Q | ASSLGQGGK DEQY |
| 5 | TSGFN G | NVLDG L | AVRGWKDGQ KLL | SGHRS | YFSET Q | ASSLGQGGK DEQY |
| 6 | TSGFN G | NVLDG L | AVRHFSDGQ KLL | SGHRS | YFSET Q | ASASGVGGK DEQY |
| 7 | TSGFN G | NVLDG L | AVRHWDGGQ KLL | SGHRS | YFSET Q | ASSLGQGGK DEQY |
| 8 | TSGFN G | NVLDG L | AVRHFSDGQ KLL | SGHRS | YFSET Q | ASSLGQGGRG TLY |
| 9 | TSGFN G | NVLDG L | AVRGWADGQ KLL | SGHRS | YFSET Q | ASSLGQGGK DEQY |
| 10 | TSGFN G | NVLDG L | AVRGWHDGQ KLL | SGHRS | YFSET Q | ASSLGQGGK DEQY |
| 11 | TSGFN G | NVLDG L | AVRGWIDGQ KLL | SGHRS | YFSET Q | ASSLGQGGK DEQY |
| 12 | TSGFN G | NVLDG L | AVRGWLDGQ KLL | SGHRS | YFSET Q | ASSLGQGGK DEQY |
| 13 | TSGFN G | NVLDG L | AVRGWMDG QKLL | SGHRS | YFSET Q | ASSLGQGGK DEQY |
| 14 | TSGFN G | NVLDG L | AVRGWNDGQ KLL | SGHRS | YFSET Q | ASSLGQGGK DEQY |
| 15 | TSGFN G | NVLDG L | AVRGWRDGQ KLL | SGHRS | YFSET Q | ASSLGQGGK DEQY |
| 16 | TSGFN G | NVLDG L | AVRGWTDGQ KLL | SGHRS | YFSET Q | ASSLGQGGK DEQY |
| 17 | TSGFN G | NVLDG L | AVRHWNNGQ KLL | SGHRS | YFSET Q | ASSLGQGGK DEQY |
| 18 | TSGFN G | NVLDG L | AVRHWQDGQ KLL | SGHRS | YFSET Q | ASSLGQGGK DEQY |
| 19 | TSGFN G | NVLDG L | AVRHWSDGQ KLL | SGHRS | YFSET Q | ASSLGQGGK DEQY |
| 20 | TSGFN G | NVLDG L | AVRHFSDGQ KLL | SGHRS | YFSET Q | ASSLGQGGRD IQY |
| 21 | TSGFN G | NVLDG L | AVRHWQRGQ KLL | SGHRS | YFSET Q | ASSLGQGGK DEQY |

In another preferred embodiment, the TCR is soluble.

In another preferred embodiment, the TCR is an αβ heterodimeric TCR and comprises an α chain TRAC constant region sequence and a β chain TRBC1 or TRBC2 constant region sequence.

In another preferred embodiment, the TCR comprises (i) all or part of a TCRα chain other than its transmembrane domain and (ii) all or part of a TCRβ chain other than its transmembrane domain, wherein both of (i) and (ii) comprise a variable domain and at least part of a constant domain of the TCR chain.

In another preferred embodiment, an artificial interchain disulfide bond is contained between an α chain constant region and a β chain constant region of the TCR.

In another preferred embodiment, one or more groups of amino acids selected from the following are substituted by cysteine residues forming the artificial interchain disulfide bond between the α chain constant region and the β chain constant region of the TCR:
Thr48 ofTRAC*01 exon 1 and Ser57 of TRBC1^{∗}01 or TRBC2*01 exon 1;
Thr45 ofTRAC*01 exon 1 and Ser77 of TRBC1^{∗}01 or TRBC2*01 exon 1;
Tyr10 ofTRAC*01 exon 1 and Ser17 of TRBC1 *01 or TRBC2*01 exon 1;
Thr45 ofTRAC*01 exon 1 and Asp59 of TRBC1 *01 or TRBC2*01 exon 1;
Ser15 ofTRAC*01 exon 1 and Glu15 of TRBC1^{∗}01 or TRBC2*01 exon 1;
Arg53 ofTRAC*01 exon 1 and Ser54 of TRBC1^{∗}01 or TRBC2*01 exon 1;
Pro89 ofTRAC*01 exon 1 and Ala19 of TRBC1^{∗}01 or TRBC2*01 exon 1;
Tyr10 ofTRAC*01 exon 1 and Glu20 of TRBC1^{∗}01 or TRBC2*01 exon 1.

In another preferred embodiment, the α chain variable domain of TCR comprises an amino acid sequence as shown in one of SEQ ID NOs: 1 and 13-29; and/or the β chain variable domain of TCR comprises an amino acid sequence as shown in one of SEQ ID NOs: 2 and 30-33.

In another preferred embodiment, the TCR is selected from the group consisting of:

| TCR No. | Sequence of α chain variable domain SEQ ID NO: | Sequence of β chain variable domain SEQ ID NO: |
|---|---|---|
| 1 | 13 | 2 |
| 2 | 14 | 2 |
| 3 | 1 | 30 |
| 4 | 15 | 2 |
| 5 | 16 | 2 |
| 6 | 1 | 31 |
| 7 | 17 | 2 |
| 8 | 1 | 32 |
| 9 | 18 | 2 |
| 10 | 19 | 2 |
| 11 | 20 | 2 |
| 12 | 21 | 2 |
| 13 | 22 | 2 |
| 14 | 23 | 2 |
| 15 | 24 | 2 |
| 16 | 25 | 2 |
| 17 | 26 | 2 |
| 18 | 27 | 2 |
| 19 | 28 | 2 |
| 20 | 1 | 33 |
| 21 | 29 | 2 |

In another preferred embodiment, the TCR is a single chain TCR.

In another preferred embodiment, the TCR is a single chain TCR consisting of an α chain variable domain and a β chain variable domain, wherein the α chain variable domain and the β chain variable domain are linked by a flexible short peptide sequence (linker).

In another preferred embodiment, a conjugate binds to an α chain and/or a β chain of the TCR at C- or N-terminal. Preferably, the conjugate is a detectable label, a therapeutic agent, a PK modified moiety or a combination thereof.

In another preferred embodiment, the therapeutic agent that binds to the TCR is an anti-CD3 antibody linked to the α or β chain of the TCR at C- or N-terminal.

In a second aspect of the present disclosure, a multivalent TCR complex is provided, which comprises at least two TCR molecules, wherein at least one TCR molecule is the TCR according to any one of the preceding claims.

In a third aspect of the present disclosure, a nucleic acid molecule is provided, which comprises a nucleic acid sequence for encoding the TCR molecule in the first aspect of the present disclosure or the multivalent TCR complex in the second aspect of the present disclosure or a complementary sequence thereof.

In a fourth aspect of the present disclosure, a vector is provided, which contains the nucleic acid molecule in the third aspect of the present disclosure.

In a fifth aspect of the present disclosure, a host cell is provided, which contains the vector in the fourth aspect of the present disclosure or has the exogenous nucleic acid molecule in the third aspect of the present disclosure integrated into a chromosome of the host cell.

In a sixth aspect of the present disclosure, an isolated cell is provided, which expresses the TCR in the first aspect of the present disclosure.

In a seventh aspect of the present disclosure, a pharmaceutical composition is provided, which contains a pharmaceutically acceptable carrier and the TCR in the first aspect of the present disclosure or the TCR complex in the second aspect of the present disclosure or the cell in the sixth aspect of the present disclosure.

In an eighth aspect of the present disclosure, a method for treating a disease is provided, which comprises administering an appropriate amount of the TCR in the first aspect of the present disclosure or the TCR complex in the second aspect of the present disclosure or the cell in the sixth aspect of the present disclosure or the pharmaceutical composition in the seventh aspect of the present disclosure to a subject in need thereof. Preferably, the disease is AFP positive tumor. More preferably, the tumor is liver cancer.

In a ninth aspect of the present disclosure, a use of the TCR in the first aspect of the present disclosure or the TCR complex in the second aspect of the present disclosure or the cell in the sixth aspect of the present disclosure is provided for preparation of a medicament for treating a tumor. Preferably, the tumor is AFP positive tumor. More preferably, the tumor is liver cancer.

In a tenth aspect of the present disclosure, a method for preparing the TCR in the first aspect of the present disclosure is provided, which comprises the steps of
(i) culturing the host cell in the fifth aspect of the present disclosure to express the T-cell receptor in the first aspect of the present disclosure;
(ii) isolating or purifying the T-cell receptor.

It is to be understood that within the scope of the present disclosure, the preceding technical features of the present disclosure and the technical features specifically described hereinafter (as in examples) may be combined with each other to constitute a new or preferred technical solution, which will not be repeated herein one by one.

### BRIEF DESCRIPTION OF DRAWINGS

FIGS. 1a and 1b show the amino acid sequences of α and β chain variable domains of a wild type TCR capable of specifically binding to KWVESIFLIF-HLAA2402 complex, respectively.
FIGS. 2a and 2b show the amino acid sequences of an α chain variable domain and a β chain variable domain of a single chain template TCR constructed in the present disclosure, respectively.
FIGS. 3a and 3b show the DNA sequences of an α chain variable domain and a β chain variable domain of a single chain template TCR constructed in the present disclosure, respectively.
FIGS. 4a and 4b show the amino acid sequence and nucleotide sequence of a linking short peptide (linker) of a single chain template TCR constructed in the present disclosure, respectively.
FIGS. 5a and 5b show the amino acid sequence and DNA sequence of a single chain template TCR constructed in the present disclosure, respectively.
FIGS. 6a and 6b show the amino acid sequences of α and β chains of a soluble reference TCR in the present disclosure, respectively.
FIGS. 7a to 7q show the amino acid sequences of an α chain variable domain of a heterodimeric TCR with high affinity for KWVESIFLIF-HLA A2402 complex, respectively, wherein the mutated residues are underlined.
FIGS. 8a to 8d show the amino acid sequences of a β chain variable domain of a heterodimeric TCR with high affinity for KWVESIFLIF-HLA A2402 complex, respectively, wherein the mutated residues are underlined.
FIGS. 9a and 9b show the extracellular amino acid sequences of α and β chains of a wild type TCR capable of specifically binding to KWVESIFLIF-HLA A2402 complex, respectively.
FIGS. 10a and 10b show the amino acid sequences of α and β chains of a wild type TCR capable of specifically binding to KWVESIFLIF-HLA A2402 complex, respectively.
FIG. 11 is a binding curve of a soluble reference TCR, i.e., a wild type TCR, to KWVESIFLIF-HLA A2402 complex.
FIG. 12 shows results of the activation function assay of effector cells transfected with a high affinity TCR of the present disclosure for T2 cells loaded with short peptides.
FIG. 13 shows results of the activation function assay of effector cells transfected with a high affinity TCR of the present disclosure for a tumor cell line.
FIG. 14 shows results of the killing function assay of effector cells transfected with a high affinity TCR of the present disclosure for T2 cells loaded with short peptides.
FIGS. 15a and 15b show results of the killing function assay (LDH assay) of effector cells transfected with a high affinity TCR of the present disclosure for a tumor cell line.
FIGS. 16a and 16b show results of the killing function assay (incuCyte assay) of effector cells transfected with a high affinity TCR of the present disclosure for a tumor cell line.

### DETAILED DESCRIPTION

Through extensive and intensive researches, the present disclosure obtains a high affinity T-cell receptor (TCR) recognizing KWVESIFLIF short peptide (derived from AFP protein) which is presented in the form of peptide-HLA A2402 complex. The high affinity TCR has a mutation in three CDRs of its α chain variable domain:
CDR1α: TSGFNG
CDR2α: NVLDGL
CDR3α: AVRHFSDGQKLL; and/or the high affinity TCR has a mutation in three CDRs of its β chain variable domain:
   CDR1β: SGHRS
   CDR2β: YFSETQ
   CDR3β: ASSLGQGGKDEQY; and after the mutation, the affinity and/or binding half-life of the TCR of the present disclosure for the above KWVESIFLIF-HLAA2402 complex is at least twice that of a wild type TCR.

Before the present disclosure is described, it is to be understood that the present disclosure is not limited to the specific methods and experimental conditions described herein, as such methods and conditions may vary. It is also to be understood that the terms used herein are only for the purpose of describing particular embodiments and are not intended to be limitative, and the scope of the present disclosure is limited by the appended claims.

Unless otherwise defined, all technical and scientific terms used herein have the same meanings as those commonly understood by those of ordinary skill in the art to which the present disclosure belongs.

Although any methods and materials similar or equivalent to those described in the present disclosure can be used in the practice or testing of the present disclosure, the preferred methods and materials are exemplified herein.

### Term

### T-cell receptor (TCR)

The TCR may be described using the International ImMunoGeneTics Information System (IMGT). A native αβ heterodimeric TCR has an α chain and a β chain. Generally speaking, each chain comprises a variable region, a junction region and a constant region, and typically the β chain also contains a short hypervariable region between the variable region and the junction region. However, the hypervariable region is often considered as part of the junction region. The junction region of the TCR is determined by the unique TRAJ and TRBJ of the IMGT, and the constant region of the TCR is determined by TRAC and TRBC of the IMGT.

Each variable region comprises three complementarity-determining regions (CDRs), CDR1, CDR2 and CDR3, which are chimeric in the framework sequence. In IMGT nomenclature, different numbers of TRAV and TRBV refer to different Vα types and Vβ types, respectively. In the IMGT system, there are the following symbols for an α chain constant domain: TRAC*01, wherein "TR" represents a TCR gene, "A" represents an α chain gene, C represents the constant region, and "*01" represents allele gene 1. There are the following symbols for a β chain constant domain: TRBC1^{∗}01 or TRBC2*01, wherein "TR" represents a TCR gene, "B" represents a β chain gene, C represents the constant region, and "*01" represents allele gene 1. The constant region of the α chain is uniquely defined, and in the form of the β chain, there are two possible constant region genes "C1" and "C2". Those skilled in the art can obtain constant region gene sequences of TCR α and β chains through the disclosed IMGT database.

The α and β chains of the TCR are generally considered as having two "domains" respectively, that is, a variable domain and a constant domain. The variable domain consists of a variable region and a junction region linked to each other. Therefore, in the description and claims of the present application, a "TCR α chain variable domain" refers to TRAV and TRAJ regions linked together. Likewise, a "TCR β chain variable domain" refers to TRBV and TRBD/TRBJ regions linked together. Three CDRs of the TCR α chain variable domain are CDR1α, CDR2α and CDR3α, respectively; and three CDRs of the TCR β chain variable domain are CDR1β, CDR2β and CDR3β, respectively. The framework sequences of TCR variable domains of the present disclosure may be of murine or human origin, preferably of human origin. The constant domain of the TCR comprises an intracellular portion, a transmembrane region and an extracellular portion.

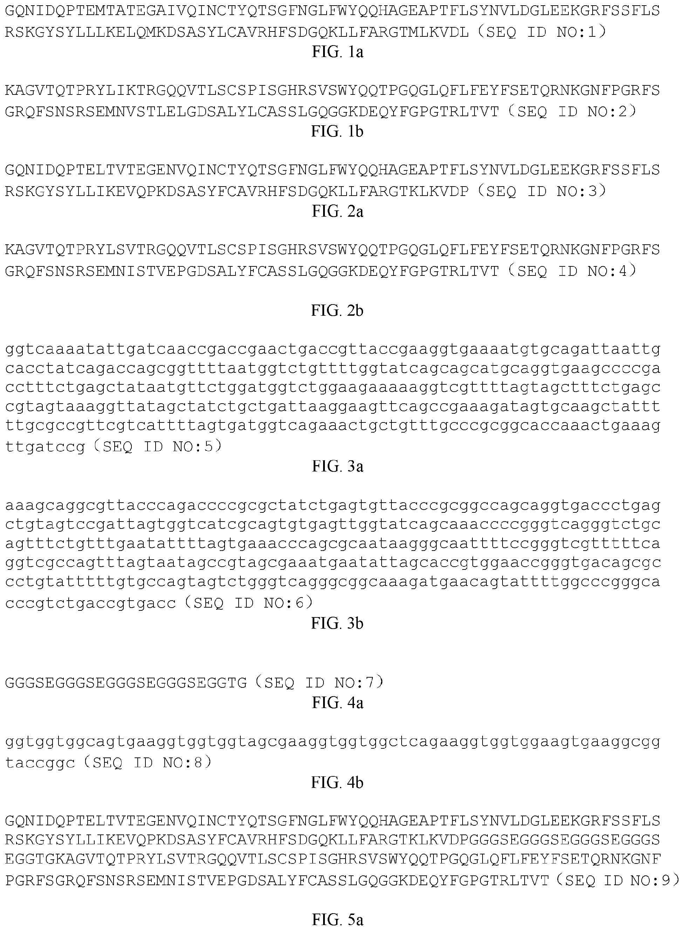

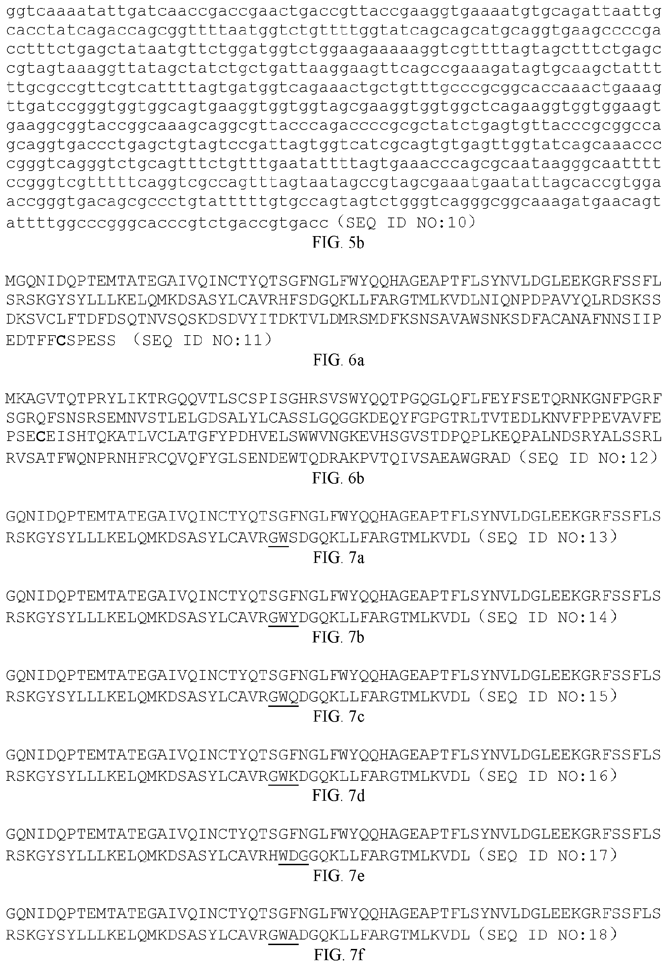

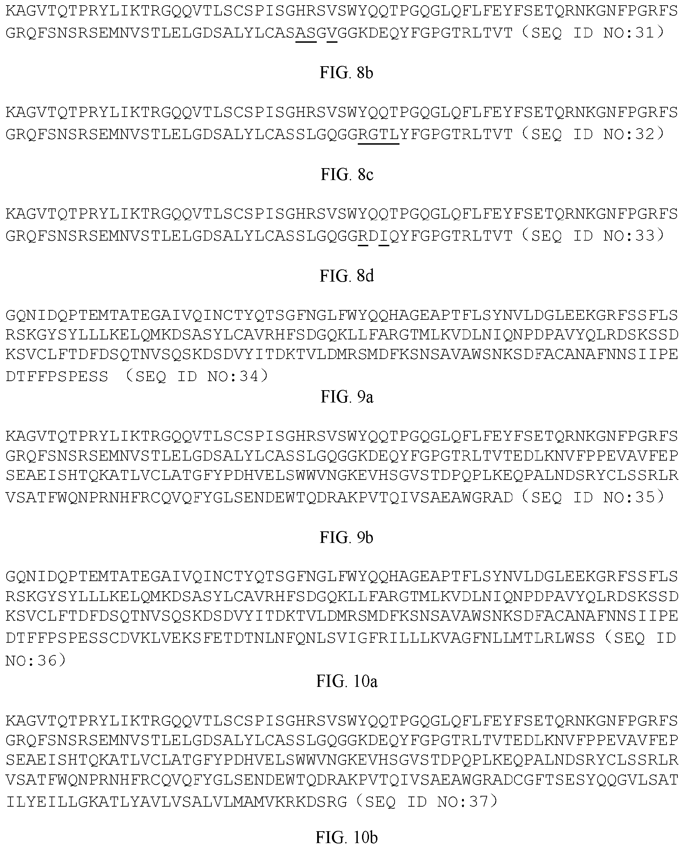

In the present disclosure, the amino acid sequences of α and β chain variable domains of a wild type TCR capable of binding to KWVESIFLIF-HLAA2402 complex are SEQ ID NO: 1 and SEQ ID NO: 2, respectively, as shown in FIGS. 1a and 1b. The amino acid sequences of α and β chains of a soluble "reference TCR" in the present disclosure are SEQ ID NO: 11 and SEQ ID NO: 12, respectively, as shown in FIGS. 6a and 6b. The extracellular amino acid sequences of α and β chains of the "wild type TCR" in the present disclosure are SEQ ID NO: 34 and SEQ ID NO: 35, respectively, as shown in FIGS. 9a and 9b. TCR sequences used in the present disclosure are of human origin. The amino acid sequences of the α chain and the β chain of the "wild type TCR" in the present disclosure are SEQ ID NO: 36 and SEQ ID NO: 37, respectively, as shown in FIGS. 10a and 10b. In the present disclosure, the terms "polypeptide of the present disclosure", "TCR of the present disclosure" and "T-cell receptor of the present disclosure" are used interchangeably.

### Natural interchain disulfide bond and artificial interchain disulfide bond

A group of disulfide bonds is present between Cα and Cβ chains in the membrane proximal region of a native TCR, which is referred to as "natural interchain disulfide bonds" in the present disclosure. In the present disclosure, an interchain covalent disulfide bond which is artificially introduced and located at a position different from the position of the natural interchain disulfide bond is referred to as "artificial interchain disulfide bond".

For convenience of description, in the present disclosure, the positions of the amino acid sequences of TRAC*01 and TRBC1*01 or TRBC2*01 are sequentially numbered from N-terminal to C-terminal. For example, the 60th amino acid in the order from N-terminal to C-terminal in TRBC1*01 or TRBC2*01 is P (proline), which may be described as Pro60 of TRBC1*01 or TRBC2*01 exon 1 in the present disclosure and may also be expressed as the amino acid at position 60 of TRBC1 *01 or TRBC2*01 exon 1. In another example, the 61st amino acid in the order from N-terminal to C-terminal in TRBC1^{∗}01 or TRBC2*01 is Q (glutamine), which may be described as Gln61 of TRBC1^{∗}01 or TRBC2*01 exon 1 in the present disclosure and may also be expressed as the amino acid at position 61 of TRBC1*01 or TRBC2*01 exon 1, and so on. In the present disclosure, the positions of the amino acid sequences of variable regions TRAV and TRBV are numbered according to the positions listed in the IMGT. For example, the position of an amino acid in TRAV is numbered as 46 in the IMGT, the amino acid is described in the present disclosure as the amino acid at position 46 of TRAV, and so on. In the present disclosure, if the positions of other amino acid sequences are specifically described, the special description shall prevail.

### Tumor

The term "tumor" refers to the inclusion of all types of cancer cell growth or carcinogenic processes, metastatic tissues or malignant transformed cells, tissues or organs, regardless of pathological type or stage of infection. Examples of tumors include, without limitation, solid tumors, soft tissue tumors and metastatic lesions. Examples of solid tumors include malignant tumors of different organ systems, such as sarcoma, lung squamous cell carcinoma and cancer. For example, infected prostate, lung, breast, lymph, gastrointestinal (e.g., colon) and genitourinary tract (e.g., kidney, epithelial cells) and pharynx. Lung squamous cell carcinoma includes malignant tumors, for example, most of colon cancer, rectal cancer, renal cell carcinoma, liver cancer, non-small cell lung cancer, small intestine cancer and esophageal cancer. Metastatic lesions of the above cancer may likewise be treated and prevented using the methods and compositions of the present disclosure.

### Detailed description of the present disclosure

As is known to all, an α chain variable domain and a β chain variable domain of a TCR each contain three CDRs, which are similar to the complementarity-determining regions of an antibody. CDR3 interacts with an antigen short peptide, and CDR1 and CDR2 interact with HLA. Therefore, the CDRs of a TCR molecule determine their interaction with an antigen short peptide-HLA complex. The amino acid sequences of the α chain variable domain and the β chain variable domain of a wild type TCR capable of binding to a complex of antigen short peptide KWVESIFLIF and HLA A2402 (that is, KWVESIFLIF-HLA A2402 complex) are SEQ ID NO: 1 and SEQ ID NO: 2, respectively, which have been discovered by the inventors for the first time. The wild type TCR has the following CDRs:
CDRs of the α chain variable domain: CDR1α: TSGFNG,
   CDR2α: NVLDGL, and
   CDR3α: AVRHFSDGQKLL;
CDRs of the β chain variable domain: CDR1β: SGHRS,
   CDR2β: YFSETQ, and
   CDR3β: ASSLGQGGKDEQY.

In the present disclosure, a high affinity TCR is obtained through mutations and screening in the preceding CDRs, where the affinity of the high affinity TCR for KWVESIFLIF-HLA A2402 complex is at least twice that of the wild type TCR for KWVESIFLIF-HLA A2402 complex.

Further, the TCR of the present disclosure is an αβ heterodimeric TCR, wherein the α chain variable domain of the TCR comprises an amino acid sequence having at least 85%, preferably at least 90%, more preferably at least 92%, most preferably at least 94% (for example, may be at least 88%, 89%, 90%, 91%, 92%, 93%, 94%, 95%, 96%, 97%, 98%, 99% of sequence homology) of sequence homology with the amino acid sequence as shown in SEQ ID NO: 1; and/or the β chain variable domain of the TCR comprises an amino acid sequence having at least 90%, preferably at least 92%, more preferably at least 94% (for example, may be at least 91%, 92%, 93%, 94%, 95%, 96%, 97%, 98%, 99% or 100% of sequence homology) of sequence homology with the amino acid sequence as shown in SEQ ID NO: 2.

Further, the TCR of the present disclosure is a single chain TCR, wherein the α chain variable domain of the TCR comprises an amino acid sequence having at least 85%, preferably at least 90%, more preferably at least 92%, most preferably at least 94% (for example, may be at least 88%, 89%, 90%, 91%, 92%, 93%, 94%, 95%, 96%, 97%, 98%, 99% of sequence homology) of sequence homology with the amino acid sequence as shown in SEQ ID NO: 3; and/or the β chain variable domain of the TCR comprises an amino acid sequence having at least 85%, preferably at least 90%, more preferably at least 92%, most preferably at least 94% (for example, may be at least 91%, 92%, 93%, 94%, 95%, 96%, 97%, 98%, 99% of sequence homology) of sequence homology with the amino acid sequence as shown in SEQ ID NO: 4.

In the present disclosure, the three CDRs of the wild type TCR α chain variable domain (SEQ ID NO: 1), i.e., CDR1, CDR2 and CDR3, are located at positions 26-31, positions 49-54 and positions 89-100 of SEQ ID NO: 1, respectively. Accordingly, amino acid residues are numbered as shown in SEQ ID NO: 1, 92H is H at position 4 of CDR3α, 93F is F at position 5 of CDR3α, 94G is G at position 6 of CDR3α, and 95D is D at position 7 of CDR3α.

The present disclosure provides a TCR having a property of binding to KWVESIFLIF-HLA A2402 complex and comprising an α chain variable domain and a β chain variable domain, wherein the TCR has a mutation in the α chain variable domain as shown in SEQ ID NO: 1, and the site of the mutated amino acid residue includes one or more of 92H, 93F, 94G and 95D, wherein the amino acid residues are numbered as shown in SEQ ID NO: 1.

Preferably, the mutated TCR α chain variable domain comprises one or more amino acid residues selected from the group consisting of: 92G; 93W; 94Y or 94Q or 94K or 94D or 94A or 94H or 94I or 94L or 94M or 94N or 94R or 94T; and 95R or 95G or 95N, wherein the amino acid residues are numbered as shown in SEQ ID NO: 1.

More specifically, in the α chain variable domain, specific forms of the mutation include one or more of H92G, F93W, G94Y/Q/K/D/A/H/I/L/M/N/R/T and D95R/G/N.

In the present disclosure, the three CDRs of the wild type TCR β chain variable domain (SEQ ID NO: 2) , i.e., CDR1, CDR2 and CDR3, are located at positions 27-31, positions 49-54 and positions 92-104 of SEQ ID NO: 2, respectively. Accordingly, amino acid residues are numbered as shown in SEQ ID NO: 2, 94S is S at position 3 of CDR3β, 95L is L at position 4 of CDR3β, 97Q is Q at position 6 of CDR3β, 100K is K at position 9 of CDR3β, 101D is D at position 10 of CDR3β, 102E is E at position 11 of CDR3β, and 103Q is Q at position 12 of CDR3β.

The present disclosure provides a TCR having a property of binding to KWVESIFLIF-HLA A2402 complex and comprising a β chain variable domain and a β chain variable domain, wherein the TCR has a mutation in the β chain variable domain as shown in SEQ ID NO: 2, and the site of the mutated amino acid residue includes one or more of 94S, 95L, 97Q, 100K, 101D, 102E and 103Q, wherein the amino acid residues are numbered as shown in SEQ ID NO: 2.

Preferably, the mutated TCR β chain variable domain comprises one or more amino acid residues selected from the group consisting of: 94A, 95S, 97V, 100R, 101M or 101G, 102Q or 102T and 103E or 103L, wherein the amino acid residues are numbered as shown in SEQ ID NO: 2.

More specifically, in the β chain variable domain, specific forms of the mutation include one or more of S94A, L95S, Q97V, K100R, D101M/G, E102Q/T and Q103E/L.

In a specific embodiment, the TCR of the present disclosure comprises the 6 CDRs in the above-shown table of CDR sequences. In a preferred embodiment, the TCR of the present disclosure comprises the 6 CDRs in each row of the above-shown table of CDR sequences.

According to the site-directed mutagenesis method well-known to those skilled in the art, Thr48 of the wild type TCRα chain constant region TRAC*01 exon 1 is mutated to cysteine and Ser57 of the β chain constant region TRBC1^{∗}01 or TRBC2*01 exon 1 is mutated to cysteine so as to obtain a reference TCR whose amino acid sequences are shown in SEQ ID NO: 11 and SEQ ID NO: 12, respectively, as shown in FIGS. 6a and 6b, wherein the mutated cysteine residues are indicated by bold letters. The above cysteine substitutions can form an artificial interchain disulfide bond between an α chain constant region and a β chain constant region of the reference TCR to form a more stable soluble TCR, so that it is easier to evaluate the binding affinity and/or binding half-life between the TCR and KWVESIFLIF-HLA A2402 complex. It will be appreciated that CDRs of the TCR variable region determine its affinity for pMHC complex so that the above cysteine substitutions in the TCR constant region will not affect the binding affinity and/or binding half-life of the TCR. Therefore, in the present disclosure, the measured binding affinity between the reference TCR and KWVESIFLIF-HLAA2402 complex is considered to be the binding affinity between the wild type TCR and KWVESIFLIF-HLA A2402 complex. Similarly, if the binding affinity between the TCR of the present disclosure and KWVESIFLIF-HLA A2402 complex is determined to be at least 10 times the binding affinity between the reference TCR and KWVESIFLIF-HLA A2402 complex, the binding affinity between the TCR of the present disclosure and KWVESIFLIF-HLAA2402 complex is at least 10 times the binding affinity between the wild type TCR and KWVESIFLIF-HLAA2402 complex.

The binding affinity (inversely proportional to a dissociation equilibrium constant K_{D}) and the binding half-life (expressed as T_{1/2}) can be determined by any suitable method. For example, the detection is performed using surface plasmon resonance technology. It is to be understood that doubling of the binding affinity of the TCR will halve K_{D}. T_{1/2} is calculated as In2 divided by a dissociation rate (K_{off}). Therefore, doubling of T_{1/2} will halve K_{off}. Preferably, the binding affinity or binding half-life of a given TCR is detected several times, for example, three or more times by using the same test protocol, and an average of the results is taken. In a preferred embodiment, the affinity of a soluble TCR is detected under the conditions of a temperature of 25 °C and a pH of 7.1-7.5 by a surface plasmon resonance (BIAcore) method in the Examples herein. The dissociation equilibrium constant K_{D} of the reference TCR to KWVESIFLIF-HLA A2402 complex is detected to be 1.76E-04M, that is, 176 µM by the method. In the present disclosure, the dissociation equilibrium constant K_{D} of the wild type TCR to KWVESIFLIF-HLA A2402 complex is also considered to be 176 µM. Since doubling of the affinity of the TCR will halve K_{D}, if the dissociation equilibrium constant K_{D} of a high affinity TCR to KWVESIFLIF-HLA A2402 complex is detected to be 1.76E-05M, that is, 17.6 µM, the affinity of the high affinity TCR for KWVESIFLIF-HLA A2402 complex is 10 times that of the wild type TCR for KWVESIFLIF-HLA A2402 complex. Those skilled in the art are familiar with the conversion relationship between K_{D} value units, that is, 1 M = 10⁶ µM, 1 µM = 1000 nM, and 1 nM = 1000 pM. In the present disclosure, the affinity of the TCR for KWVESIFLIF-HLAA2402 complex is at least twice, preferably at least 10 times; more preferably at least 100 times that of the wild type TCR.

Mutations can be carried out by any suitable method including, but not limited to, those based on the polymerase chain reaction (PCR), restriction enzyme-based cloning or a ligation-independent cloning (LIC) method. These methods are described in detail in many standard molecular biology textbooks. More details about polymerase chain reaction (PCR) mutagenesis and restriction enzyme-based cloning can be found in Sambrook and Russell, (2001) Molecular Cloning-A Laboratory Manual (Third Edition) CSHL Publishing house. More information about the LIC method can be found in Rashtchian, (1995) Curr Opin Biotechnol 6(1): 30-6.

A method for producing the TCR of the present disclosure may be, but is not limited to, screening a TCR having high affinity for KWVESIFLIF-HLA-A2402 complex from a diverse library of phage particles displaying such TCRs, as described in the literature (Li, et al. (2005) Nature Biotech 23(3): 349-354).

It will be appreciated that genes expressing amino acids of α and β chain variable domains of the wild type TCR or genes expressing amino acids of α and β chain variable domains of a slightly modified wild type TCR can both be used for preparing template TCRs. Changes necessary to produce the high affinity TCR of the present disclosure are then introduced into the DNA encoding the variable domain of the template TCR.

The high affinity TCR of the present disclosure comprises one of amino acid sequences of the α chain variable domain as shown in SEQ ID NOs: 1 and 13-29; and/or one of amino acid sequences of the β chain variable domain as shown in SEQ ID NOs: 2 and 30-33. In the present disclosure, the amino acid sequences of the α chain variable domain and the β chain variable domain which form the heterodimeric TCR molecule are preferably selected from Table 1.

**Table 1**

| TCR No. | Sequence of α chain variable domain SEQ ID NO: | Sequence of β chain variable domain SEQ ID NO: |
|---|---|---|
| 1 | 13 | 2 |
| 2 | 14 | 2 |
| 3 | 1 | 30 |
| 4 | 15 | 2 |
| 5 | 16 | 2 |
| 6 | 1 | 31 |
| 7 | 17 | 2 |
| 8 | 1 | 32 |
| 9 | 18 | 2 |
| 10 | 19 | 2 |
| 11 | 20 | 2 |
| 12 | 21 | 2 |
| 13 | 22 | 2 |
| 14 | 23 | 2 |
| 15 | 24 | 2 |
| 16 | 25 | 2 |
| 17 | 26 | 2 |
| 18 | 27 | 2 |
| 19 | 28 | 2 |
| 20 | 1 | 33 |
| 21 | 29 | 2 |

Based on the object of the present disclosure, the TCR of the present disclosure is a moiety having at least one TCR α and/or TCR β chain variable domain. Such TCRs generally comprise both the TCR α chain variable domain and the TCR β chain variable domain. They may be αβ heterodimers or in a single chain form or any other stable forms. In adoptive immunotherapy, the full length chain of the αβ heterodimeric TCR (including the cytoplasmic and transmembrane domains) can be transfected. The TCR of the present disclosure can be used as a targeting agent for delivering a therapeutic agent to an antigen-presenting cell or be used in combination with other molecules to prepare a bifunctional polypeptide to direct effector cells when the TCR is preferably in a soluble form.

As for stability, it is disclosed in the existing art that a soluble and stable TCR molecule can be obtained by introducing an artificial interchain disulfide bond between the α and β chain constant domains of a TCR, as described in PCT/CN2015/093806. Therefore, the TCR of the present disclosure may be a TCR with an artificial interchain disulfide bond introduced between the residues of its α and β chain constant domains. Cysteine residues form an artificial interchain disulfide bond between the α and β chain constant domains of the TCR. Cysteine residues can replace other amino acid residue at suitable positions of a native TCR to form an artificial interchain disulfide bond. For example, Thr48 of TRAC*01 exon 1 and Ser57 of TRBC1^{∗}01 or TRBC2*01 exon 1 can be replaced to form a disulfide bond. Other sites for introducing a cysteine residue to form a disulfide bond may be: Thr45 of TRAC^{∗}01 exon 1 and Ser77 of TRBC1^{∗}01 or TRBC2*01 exon 1; Tyr10 ofTRAC*01 exon 1 and Ser17 of TRBC1^{∗}01 or TRBC2*01 exon 1; Thr45 ofTRAC*01 exon 1 and Asp59 of TRBC1^{∗}01 or TRBC2*01 exon 1; Ser15 ofTRAC*01 exon 1 and Glu15 of TRBC1^{∗}01 or TRBC2*01 exon 1; Arg53 ofTRAC*01 exon 1 and Ser54 of TRBC1*01 or TRBC2*01 exon 1; Pro89 of TRAC*01 exon 1 and Ala19 of TRBC1^{∗}01 or TRBC2*01 exon 1; or Tyr10 ofTRAC*01 exon 1 and Glu20 of TRBC1^{∗}01 or TRBC2*01 exon 1. That is, cysteine residues replace any group of the above-mentioned sites in α and β chain constant domains. A maximum of 15, or a maximum of 10, or a maximum of 8 or fewer amino acids may be truncated at one or more C-termini of the constant domain of the TCR of the present disclosure such that it does not include cysteine residues to achieve the purpose of deleting a natural interchain disulfide bond, or the cysteine residues forming a natural interchain disulfide bond can also be mutated to another amino acid for achieving the above purpose.

As described above, the TCR of the present disclosure may comprise an artificial interchain disulfide bond introduced between residues of its α and β chain constant domains. It is to be noted that the introduced artificial disulfide bond as described above can be contained or not contained between the constant domains, and the TCR of the present disclosure may contain a TRAC constant domain sequence and a TRBC1 or TRBC2 constant domain sequence. The TRAC constant domain sequence and the TRBC1 or TRBC2 constant domain sequence of the TCR can be linked by a natural interchain disulfide bond present in the TCR.

Additionally, as for stability, it is also disclosed in a patent literature PCT/CN2016/077680 that the introduction of an artificial interchain disulfide bond between the α chain variable region and the β chain constant region of a TCR can significantly improve the stability of the TCR. Therefore, an artificial interchain disulfide bond may be contained between an α chain variable region and a β chain constant region of the high affinity TCR of the present disclosure. Specifically, cysteine residues forming an artificial interchain disulfide bond between the α chain variable region and the β chain constant region of the TCR replace: an amino acid at position 46 of TRAV and an amino acid at position 60 of TRBC1^{∗}01 or TRBC2*01 exon 1; an amino acid at position 47 of TRAV and an amino acid at position 61 of TRBC1^{∗}01 or TRBC2*01 exon 1; an amino acid at position 46 of TRAV and an amino acid at position 61 of TRBC1^{∗}01 or TRBC2*01 exon 1; or an amino acid at position 47 of TRAV and an amino acid at position 60 of TRBC1 *01 or TRBC2*01 exon 1. Preferably, such a TCR may comprise (i) all or part of a TCR α chain other than its transmembrane domain and (ii) all or part of a TCR β chain other than its transmembrane domain, wherein both of (i) and (ii) comprise a variable domain and at least part of a constant domain of the TCR chain, and the α chain and the β chain form a heterodimer. More preferably, such TCR may comprise an α chain variable domain, a β chain variable domain and all or part of a β chain constant domain other than the transmembrane domain, which, however, does not comprise an α chain constant domain, and the α chain variable domain and the β chain of the TCR form a heterodimer.

As for stability, in another aspect, the TCR of the present disclosure also includes a TCR having a mutation in its hydrophobic core region, and these mutations in the hydrophobic core region are preferably mutations capable of increasing the stability of the TCR of the present disclosure, as described in WO 2014/206304. Such a TCR can have mutations at the following positions in the variable domain hydrophobic core: (α and/or β chain) variable region amino acids at positions 11, 13, 19, 21, 53, 76, 89, 91, 94, and/or α chain J gene (TRAJ) short peptide amino acids at reciprocal positions 3, 5, 7, and/or β chain J gene (TRBJ) short peptide amino acids at reciprocal positions 2, 4, 6, wherein the positions in an amino acid sequence are numbered according to the position numbers listed in the International ImMunoGeneTics Information System (IMGT). Those skilled in the art know the preceding IMGT and can obtain the position numbers of amino acid residues of different TCRs in the IMGT based on the database.

More specifically, in the present disclosure, a TCR having a mutation in its hydrophobic core region may be a high-stability single chain TCR consisting of TCR α and β chain variable domains linked by a flexible peptide chain. CDRs in the variable region of the TCR determine the affinity of the TCR for a short peptide-HLA complex, and mutations in a hydrophobic core can increase the stability of the TCR without affecting the affinity of the TCR for the short peptide-HLA complex. It is to be noted that the flexible peptide chain in the present disclosure may be any peptide chain suitable for linking TCR α and β chain variable domains. A template chain constructed in Example 1 of the present disclosure for screening high affinity TCRs is the preceding high-stability single chain TCR containing mutations in the hydrophobic core. The affinity between a TCR and KWVESIFLIF-HLA-A2402 complex can be more easily evaluated by using a TCR with higher stability.

The CDRs of an α chain variable domain and a β chain variable domain of the single chain template TCR are identical to the CDRs of the wild type TCR. That is, three CDRs of the α chain variable domain are CDR1α: TSGFNG, CDR2α: NVLDGL, and CDR3α: AVRHFSDGQKLL, respectively, and three CDRs of the β chain variable domain are CDR1β: SGHRS, CDR2β: YFSETQ, and CDR3β: ASSLGQGGKDEQY, respectively. The amino acid sequence (SEQ ID NO: 9) and the nucleotide sequence (SEQ ID NO: 10) of the single chain template TCR are shown in FIGS. 5a and 5b, respectively, thereby screening a single chain TCR consisting of an α chain variable domain and a β chain variable domain and having high affinity for KWVESIFLIF-HLA A2402 complex.

In the present disclosure, an αβ heterodimer having high affinity for KWVESIFLIF-HLA-A2402 complex is obtained by transferring the CDRs of α and β chain variable domains of the screened high affinity single chain TCR to the corresponding positions of the α chain variable domain (SEQ ID NO: 1) and the β chain variable domain (SEQ ID NO: 2) of the wild type TCR.

The TCR of the present disclosure can also be provided in the form of a multivalent complex. The multivalent TCR complex of the present disclosure comprises a polymer formed by combining two, three, four or more TCRs of the present disclosure, for example, a tetrameric domain of p53 can be used to produce a tetramer. Alternatively, multiple TCRs of the present disclosure can be combined with another molecule to form a complex. The TCR complex of the present disclosure can be used to track or target cells that present a particular antigen *in vitro* or *in vivo* or produce intermediates of other multivalent TCR complexes with such uses.

The TCR of the present disclosure may be used alone or combined with a conjugate in a covalent manner or another manner, preferably in the covalent manner. The conjugate includes a detectable label (for diagnostic purposes, wherein the TCR is used to detect the presence of a cell presenting KWVESIFLIF-HLA-A2402 complex), a therapeutic agent, a protein kinase (PK) modified moiety or any combination of the preceding substances.

Detectable labels for diagnostic purposes include, but are not limited to, fluorescent or luminescent labels, radioactive labels, MRI (magnetic resonance imaging) or CT (electron computed X-Ray tomography technology) contrast agents or enzymes capable of producing detectable products.

Therapeutic agents that can be combined with or coupled to the TCR of the present disclosure include, but are not limited to: 1. radionuclides (Koppe et al., 2005, Cancer metastasis reviews, 24, 539); 2. biotoxin (Chaudhary et al., 1989, Nature, 339, 394; Epel et al., 2002, Cancer Immunology and Immunotherapy, 51, 565); 3. cytokines such as IL-2 (Gillies et al., 1992, Proceedings of the National Academy of Sciences of the United States of America (PNAS), 89, 1428; Card et al., 2004, Cancer Immunology and Immunotherapy, 53, 345; Halin et al., 2003, Cancer Research, 63, 3202); 4. antibody Fc fragments (Mosquera et al., 2005, the Journal of Immunology, 174, 4381); 5. antibody scFv fragments (Zhu et al., 1995, International Journal of Cancer, 62, 319); 6. gold nanoparticles/nanorods (Lapotko et al., 2005, Cancer letters, 239, 36; Huang et al., 2006, Journal of the American Chemical Society 128, 2115); 7. viral particles (Peng et al., 2004, Gene therapy, 11, 1234); 8. liposomes (Mamot et al., 2005, Cancer research, 65, 11631); 9. nanomagnetic particles; 10. prodrug activating enzymes (e.g., DT-diaphorase (DTD) or biphenyl hydrolase-like protein (BPHL); 11. chemotherapeutic agents (e.g., cisplatin) or any form of nanoparticles, and the like.

An antibody binding to the TCR of the present disclosure or a fragment thereof includes an anti-T cell or an NK-cell determining antibody, such as an anti-CD3 or anti-CD28 or anti-CD16 antibody. The above antibody or a fragment thereof binds to the TCR, which can direct effector cells to better target target cells. In a preferred embodiment, the TCR of the present disclosure binds to an anti-CD3 antibody or a functional fragment or variant thereof. Specifically, a fusion molecule of the TCR of the present disclosure and an anti-CD3 single chain antibody comprises a TCR α chain variable domain whose amino acid sequence is one of SEQ ID NOs: 1 and 3-29; and/or a TCR β chain variable domain whose amino acid sequence is one of SEQ ID NOs: 2 and 30-33.

The present disclosure further relates to a nucleic acid molecule encoding the TCR of the present disclosure. The nucleic acid molecule of the present disclosure may be in the form of DNA or RNA. DNA may be a coding strand or a non-coding strand. For example, a nucleic acid sequence encoding the TCR of the present disclosure may be the same as the nucleic acid sequence shown in the figures of the present disclosure or a degenerate variant thereof. By way of example, the "degenerate variant", as used herein, refers to a nucleic acid sequence which encodes a protein with a sequence of SEQ ID NO: 3 but is different from the sequence of SEQ ID NO: 5.

The full length sequence of the nucleic acid molecule of the present disclosure or a fragment thereof may generally be obtained by, but not limited to, a PCR amplification method, a recombination method or an artificial synthesis method. At present, it has been possible to obtain a DNA sequence encoding the TCR (or a fragment thereof, or a derivative thereof) of the present disclosure completely by chemical synthesis. Then, the DNA sequence can be introduced into various existing DNA molecules (or such as vectors) and cells known in the art.

The present disclosure further relates to a vector comprising the nucleic acid molecule of the present disclosure as well as a host cell genetically engineered using the vector or coding sequence of the present disclosure.

The present disclosure further encompasses an isolated cell, particularly a T cell, which express the TCR of the present disclosure. There are many methods suitable for T cell transfection with DNA or RNA encoding the high affinity TCR of the present disclosure (e.g., Robbins et al., (2008) J. Immunol. 180: 6116-6131). T cells expressing the high affinity TCR of the present disclosure can be used for adoptive immunotherapy. Those skilled in the art can know many suitable methods for adoptive therapy (e.g., Rosenberg et al., (2008) Nat Rev Cancer 8(4): 299-308).

The present disclosure further provides a pharmaceutical composition, which comprises a pharmaceutically acceptable carrier and the TCR of the present disclosure or the TCR complex of the present disclosure or the cell presenting the TCR of the present disclosure.

The present disclosure further provides a method for treating a disease, which comprises administering an appropriate amount of the TCR of the present disclosure or the TCR complex of the present disclosure or the cell presenting the TCR of the present disclosure or the pharmaceutical composition of the present disclosure to a subject in need thereof.

It is to be understood that amino acid names herein are identified by internationally accepted single English letters, and the corresponding three-letter abbreviated names of amino acids are: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), Val (V).

In the present disclosure, Pro60 or 60P represents proline at position 60. Further, regarding the expression of the specific form of a mutation in the present disclosure, for example, "H92G" represents that H at position 93 is substituted with G. Similarly, "D95R/G" represents that D at position 95 is substituted with R or G. The rest can be done in the same manner.

In the art, when an amino acid with similar properties is used for substitution, the function of a protein is generally not changed. The addition of one amino acid or multiple amino acids at C-terminal and/or N-terminal generally will not change the structure and function of the protein. Therefore, the TCR of the present disclosure further includes a TCR wherein up to 5, preferably up to 3, more preferably up to 2, most preferably 1 amino acid (especially an amino acid located outside CDRs) of the TCR of the present disclosure is replaced with an amino acid with similar properties and which can still maintain its function.

The present disclosure further includes a TCR slightly modified from the TCR of the present disclosure. The form of a modification (generally without altering a primary structure) includes chemically derived forms of the TCR of the present disclosure, such as acetylation or carboxylation. Modifications also include glycosylation, such as those TCRs produced through glycosylation in the synthesis and processing steps or in the further processing step of the TCR of the present disclosure. Such modification can be accomplished by exposing the TCR to an enzyme for glycosylation (such as a mammalian glycosylation enzyme or a deglycosylation enzyme). The form of the modification further includes sequences having phosphorylated amino acid residues (such as phosphotyrosine, phosphoserine and phosphothreonine). Further included are TCRs that have been modified to enhance their antiproteolytic properties or optimize solubility properties.

The TCR or TCR complex of the present disclosure or the T cell transfected with the TCR of the present disclosure may be provided in a pharmaceutical composition together with a pharmaceutically acceptable carrier. The TCR, multivalent TCR complex or cell of the present disclosure is typically provided as part of a sterile pharmaceutical composition which typically comprises a pharmaceutically acceptable carrier. The pharmaceutical composition may be in any suitable form (depending on the desired method for administration to a patient). The pharmaceutical composition may be provided in a unit dosage form, generally provided in a sealed container and may be provided as part of a kit. Such kits include instructions (which are not necessary). The pharmaceutical composition may include multiple unit dosage forms.

Furthermore, the TCR of the present disclosure may be used alone or in combination with other therapeutic agents (e.g., formulated in the same pharmaceutical composition).

The pharmaceutical composition may also contain a pharmaceutically acceptable carrier. The term "pharmaceutically acceptable carrier" refers to a carrier for administration of a therapeutic agent. The term refers to such pharmaceutical carriers which do not induce the production of antibodies harmful to an individual receiving the composition and which are not excessively toxic after administration. These carriers are well known to those skilled in the art. A full discussion of pharmaceutically acceptable excipients can be found in Remington's Pharmaceutical Sciences (Mack Pub. Co., N. J. 1991). Such carriers include, but are not limited to, saline, buffer, dextrose, water, glycerol, ethanol, adjuvants and combinations thereof.

The pharmaceutically acceptable carrier in the therapeutic composition may contain a liquid such as water, saline, glycerol and ethanol. In addition, auxiliary substances such as wetting or emulsifying agents and pH buffering substances may also be present in these carriers.

Generally, the therapeutic compositions may be formulated as an injectable, such as liquid solutions or suspensions; and may also be formulated as solid forms such as liquid carriers, which may be suitable for being formulated in solutions or suspensions prior to injection.

Once the composition of the present disclosure is formulated, it may be administered by conventional routes including, but not limited to, intraocular, intramuscular, intravenous, subcutaneous, intradermal or topical administration, preferably parenteral administration including subcutaneous, intramuscular or intravenous administration. A subject to be prevented or treated may be an animal, especially a human.

When the pharmaceutical composition of the present disclosure is used for actual treatment, pharmaceutical compositions of various dosage forms may be employed depending on uses, preferably, an injection, an oral preparation or the like.

These pharmaceutical compositions may be formulated by being mixed, diluted or dissolved by conventional methods and, occasionally, be added with suitable pharmaceutical additives such as excipients, disintegrating agents, binders, lubricants, diluents, buffers, isotonicities, preservatives, wetting agents, emulsifiers, dispersing agents, stabilizers and co-solvents, and the formulation process may be carried out in a customary manner depending on the dosage form.

The pharmaceutical composition of the present disclosure may also be administered in the form of a sustained release preparation. For example, the TCR of the present disclosure may be incorporated into a pill or microcapsule with a sustained release polymer as a carrier and then the pill or microcapsule is surgically implanted into the tissue to be treated. Examples of the sustained release polymer include ethylene-vinyl acetate copolymer, polyhydrometaacrylate, polyacrylamide, polyvinylpyrrolidone, methylcellulose, lactic acid polymer, lactic acid-glycolic acid copolymer or the like, preferably a biodegradable polymer such as lactic acid polymer and lactic acid-glycolic acid copolymer.

When the pharmaceutical composition of the present disclosure is used for actual treatment, the amount of the TCR or TCR complex of the present disclosure or the cell presenting the TCR of the present disclosure as an active ingredient may be reasonably determined by a doctor based on the body weight, age, sex and degree of symptoms of each patient to be treated.

### Main advantages of the present disclosure:

(1) The affinity and/or binding half-life of the high affinity TCR of the present disclosure for KWVESIFLIF-HLA-A2402 complex is at least twice, preferably at least 10 times, more preferably at least 100 times that of the wild type TCR.
(2) The high affinity TCR of the present disclosure can specifically bind to KWVESIFLIF-HLA A2402, and cells transfected with the high affinity TCR of the present disclosure can be specifically activated and proliferated.
(3) Effector cells transfected with the high affinity TCR of the present disclosure exhibit a strong killing effect on target cells.

The present disclosure is further illustrated by the following specific examples. It is to be understood that these examples are used only for the purpose of illustration and are not intended to limit the scope of the present disclosure. Experimental methods in the following examples which do not specify specific conditions are generally performed under conventional conditions, for example, conditions described in Sambrook and Russell et al., Molecular Cloning-A Laboratory Manual (Third Edition) (2001) CSHL Publishing house or under conditions recommended by the manufacturer. Percentages and parts are by weight unless otherwise stated.

### Materials and Methods

The experimental materials used in the examples of the present disclosure may be commercially available, unless otherwise specified, wherein *E*. *coli* DH5α was purchased from Tiangen, *E. coli* BL21 (DE3) was purchased from Tiangen, *E. coli* Tuner (DE3) was purchased from Novagen, and plasmid pET28a was purchased from Novagen.

### Example 1 Generation of stable single chain TCR template chains with mutations in the hydrophobic core

In the present disclosure, a stable single chain TCR molecule consisting of TCR α and β chain variable domains linked by a flexible short peptide (linker) was constructed by a site-directed mutagenesis method according to a patent literature WO2014/206304, where the amino acid sequence and DNA sequence thereof are SEQ ID NO: 9 and SEQ ID NO: 10, respectively, as shown in FIGS. 5a and 5b. The single chain TCR molecule was used as a template for screening a high affinity TCR molecule. The amino acid sequences of an α variable domain (SEQ ID NO: 3) and a β variable domain (SEQ ID NO: 4) of the template chain are shown in FIGS. 2a and 2b; the corresponding DNA sequences are SEQ ID NO: 5 and SEQ ID NO: 6, respectively, as shown in FIGS. 3a and 3b; and the amino acid sequence and DNA sequence of the flexible short peptide (linker) are SEQ ID NO: 7 and SEQ ID NO: 8, respectively, as shown in FIGS. 4a and 4b.

The target gene carrying the template chain was digested with NcoI and NotI and ligated with pET28a vector digested with NcoI and NotI. The ligation product was transferred into *E. coli* DH5α, plated on a kanamycin-containing LB plate, inverted and cultured at 37 °C overnight, and the positive clones were picked for PCR screening. Positive recombinants were sequenced to determine the correct sequence and the recombinant plasmid was extracted and transferred into *E. coli* BL21 (DE3) for expression.

### Example 2 Expression, refolding and purification of the stable single chain TCR constructed in Example 1

All of BL21(DE 3) colonies containing the recombinant plasmid pET28a-template chain prepared in Example 1 were inoculated into the LB medium containing kanamycin and cultured at 37 °C until OD₆₀₀ was 0.6-0.8. IPTGwas added to a final concentration of 0.5 mM and cultured at 37 °C for another 4 h. The cell pellets were harvested by centrifugation at 5000 rpm for 15 min, and the cell pellets were lysed with Bugbuster Master Mix (Merck). The inclusion bodies were recovered by centrifugation at 6000 rpm for 15 min, followed by washing with Bugbuster (Merck) to remove cell debris and membrane components. The inclusion bodies were collected by centrifugation at 6000 rpm for 15 min. The inclusion bodies were dissolved in a buffer (20 mM Tris-HCl pH 8.0, 8 M urea), and the insoluble substances were removed by high-speed centrifugation. The supernatant was quantitatively determined by the BCA method and then dispensed and stored at -80 °C until use.

To 5 mg of dissolved single-chain TCR inclusion body protein, 2.5 mL of buffer (6 M Gua-HCl, 50 mM Tris-HCl pH 8.1, 100 mM NaCl, 10 mM EDTA) was added, and then DTT was added to a final concentration of 10 mM and incubated at 37 °C for 30 min. The single chain TCR as treated above was added dropwise to 125 mL of refolding buffer (100 mM Tris-HCl pH 8.1, 0.4 M L-arginine, 5 M urea, 2 mM EDTA, 6.5 mM β-mercapthoethylamine, 1.87 mM cystamine) with a syringe and stirred at 4 °C for 10 min. Then the refolded solution was loaded into a cellulose membrane dialysis bag with a cut-off of 4 kDa, and the dialysis bag was placed in 1 L of precooled water and stirred slowly at 4 °C overnight. After 17 h, the dialysis liquid was changed to 1 L of pre-chilled buffer (20 mM Tris-HCl pH 8.0) and dialysis was continued for 8 h at 4 °C. The dialysis liquid was then replaced with the same fresh buffer and dialysis was continued overnight. After 17 h, the sample was filtered through a 0.45 µm filter, vacuum degassed and purified through an anion-exchange column (HiTrap Q HP, GE Healthcare) with a linear gradient elution of 0-1 M NaCl prepared with 20 mM Tris-HCl pH 8.0. The collected fractions were subjected to SDS-PAGE analysis, the fractions containing the single chain TCR were concentrated and further purified by a gel filtration column (Superdex 75 10/300, GE Healthcare), and the target fractions were also subjected to the SDS-PAGE analysis.

The eluted fractions for BIAcore analysis were further tested using gel filtration for purity. The conditions were a chromatographic column Agilent Bio SEC-3 (300 A, ϕ7.8×300 mm), a mobile phase 150 mM phosphate buffer, a flowrate of 0.5 mL/min, a column temperature of 25 °C, and an ultraviolet detection wavelength of 214 nm.

### Example 3 Binding characterization

### BIAcore analysis

The binding activity of a TCR molecule to KWVESIFLIF-HLA-A2402 complex was detected using the BIAcore T200 real-time analysis system. An anti-streptavidin antibody (GenScript) was added to a coupling buffer (10 mM sodium acetate buffer, pH 4.77), and then the antibody passed through a CM5 chip pre-activated with EDC and NHS to immobilize the antibody on the surface of the chip. The unreacted activated surface was finally blocked with a solution of ethanolamine in hydrochloric acid to complete the coupling process at a coupling level of about 15000 RU. The conditions were a temperature of 25 °C and a pH of 7.1-7.5.

Streptavidin with a low concentration flowed over the surface of the antibody-coated chip, and then KWVESIFLIF-HLA-A2402 complex flowed through the detection channel with another channel as a reference channel. 0.05 mM biotin flowed over the chip for 2 min at a flowrate of 10 µL/min, thereby blocking the remaining binding sites for streptavidin. The affinity was determined by a single-cycle kinetic analysis. The TCR was diluted to several different concentrations with HEPES-EP buffer (10 mM HEPES, 150 mM NaCl, 3 mM EDTA, 0.005% P20, pH 7.4) and flowed over the surface of the chip in sequence at a flowrate of 30 µL/min, with a binding time of 120s per injection. After the last injection, the chip was placed for dissociation for 600s. At the end of each round of assay, the chip was regenerated with 10 mM Gly-HCl, pH 1.75. Kinetic parameters were calculated using BIAcore Evaluation software.

The preparation process of the preceding KWVESIFLIF-HLA-A2402 complex is described below.
a. Purification: 100 mL of *E. coli* bacteria liquid induced to express heavy or light chains was collected and centrifuged at 8000 g for 10 min at 4 °C, and the bacteria cells were washed once with 10 mL of PBS and then vigorously shaken in 5 mL of BugBuster Master Mix Extraction Reagents (Merck) for resuspending the bacteria cells. The suspension was rotated and incubated for 20 min at room temperature and then centrifuged at 6000 g for 15 min at 4 °C. The supernatant was discarded to collect inclusion bodies.
   The above inclusion bodies were resuspended in 5 mL of BugBuster Master Mix, rotated and incubated at room temperature for 5 min. 30 mL of BugBuster diluted 10 times was added, mixed and centrifuged at 6000 g for 15 min at 4 °C. The supernatant was discarded, and 30 mL of BugBuster diluted 10 times was added to resuspend the inclusion bodies, mixed, centrifuged at 6000 g at 4 °C for 15 min, and repeated twice. 30 mL of 20 mM Tris-HCl pH 8.0 was added to resuspend the inclusion bodies, mixed and centrifuged at 6000 g at 4 °C for 15 min. Finally, the inclusion bodies were dissolved in 20 mM Tris-HCl 8M urea. The purity of the inclusion bodies was determined by SDS-PAGE and the concentration was measured by the BCAkit.
b. Refolding: The synthesized short peptide KWVESIFLIF (Beijing SBS Genetech Co., Ltd.) was dissolved in DMSO to a concentration of 20 mg/mL. Inclusion bodies of light and heavy chains were solubilized in 8 M urea, 20 mM Tris pH 8.0, 10 mM DTT and further denatured by adding 3 M guanidine hydrochloride, 10 mM sodium acetate, 10 mM EDTA before refolding. KWVESIFLIF peptide was added to a refolding buffer (0.4 M L-arginine, 100 mM Tris pH 8.3, 2 mM EDTA, 0.5 mM oxidized glutathione, 5 mM reduced glutathione, 0.2 mM PMSF, cooled to 4 °C ) at 25 mg/L (final concentration). Then, 20 mg/L of light chain and 90 mg/L of heavy chain (final concentration, heavy chains were added in three portions, 8 h/portion) were successively added and refolded at 4 °C for at least three days to completion of refolding, and SDS-PAGE was used to confirm refolding.
c. Purification upon refolding: The refolding buffer was replaced with 10 volumes of 20 mM Tris pH 8.0 for dialysis, and the buffer was replaced at least twice to fully reduce the ionic strength of the solution. After dialysis, the protein solution was filtered through a 0.45 µm cellulose acetate filter and loaded onto a HiTrap Q HP (GE, General Electric Company) anion-exchange column (5 mL bed volume). The protein was eluted with a linear gradient of 0-400 mM NaCl prepared in 20 mM Tris pH 8.0 using Akta Purifier (GE, General Electric Company), and pMHC was eluted at approximately 250 mM NaCl. All peak fractions were collected and the purity thereof was detected by SDS-PAGE.
d. Biotinylation: Purified pMHC molecules were concentrated with a Millipore ultrafiltration tube while the buffer was replaced with 20 mM Tris pH 8.0. Then, biotinylation reagents 0.05 M Bicine pH 8.3, 10 mM ATP, 10 mM MgOAc, 50 µM D-Biotin, 100 µg/ml BirA enzyme (GST-BirA) were added. The resulting mixture was incubated at room temperature overnight, and SDS-PAGE was used to detect the completion of biotinylation.
e. Purification of the biotinylated complex: The biotinylated and labeled pMHC molecules were concentrated to 1 mL with the Millipore ultrafiltration tube. The biotinylated pMHC was purified by gel permeation chromatography using an Akta Purifier (GE, General Electric Company). 1 mL of concentrated biotinylated pMHC molecules was loaded on a HiPrep^{™} 16/60 S200 HR column (GE, General Electric Company) pre-equilibrated with filtered PBS and eluted with PBS at a flowrate of 1 mL/min. The biotinylated pMHC molecules were eluted as a single peak at about 55 mL. The protein-containing fractions were combined and concentrated with the Millipore ultrafiltration tube. The concentration of proteins was determined by the BCA method (Thermo), a protease inhibitor cocktail (Roche) was added, and the biotinylated pMHC molecules were dispensed and stored at -80 °C.

### Example 4 Generation of a high affinity TCR

Phage display technology is a means to generate high affinity TCR variant libraries for screening high affinity variants. The TCR phage display and screening method described by Li et al. ((2005) Nature Biotech 23(3): 349-354) was applied to the single chain TCR template in Example 1. A library of high affinity TCRs was established by mutating CDRs of the template chain and panned. After several rounds of panning, the phage library can specifically bind to the corresponding antigen, a monoclone was picked, and an analysis was performed.

The mutations in CDRs of the screened high affinity single chain TCRs were introduced into the corresponding sites of the variable domains of the αβ heterodimeric TCR, and the affinity of the αβ heterodimeric TCR for KWVESIFLIF-HLA-A2402 complex was detected by BIAcore. The mutated sites of high affinity were introduced into the above CDRs by a site-directed mutagenesis method well-known to those skilled in the art. The amino acid sequences of α and β chain variable domains of the above wild type TCR are shown in FIGS. 1a (SEQ ID NO: 1) and 1b (SEQ ID NO: 2), respectively.

It is to be noted that to obtain a more stable soluble TCR for easier evaluation of the binding affinity and/or binding half-life between the TCR and KWVESIFLIF-HLAA2402 complex, the αβ heterodimeric TCR may be a TCR in which a cysteine residue is respectively introduced into α and β chain constant regions to form an artificial interchain disulfide bond. In this example, the amino acid sequences of TCR α and β chains after the introduction of the cysteine residue are shown in FIGS. 6a (SEQ ID NO: 11) and 6b (SEQ ID NO: 12), where the introduced cysteine residues are indicated by bold letters.

According to standard methods described in Molecular Cloning-A Laboratory Manual (Third Edition, Sambrook and Russell), genes of extracellular sequences of TCR α and β chains to be expressed are synthesized and inserted into an expression vector pET28a+ (Novagene) in which the upstream and downstream cloning sites are NcoI and NotI, respectively. Mutations in the CDRs are introduced by overlap PCR well-known to those skilled in the art. The inserted fragment was sequenced to confirm that it was correct.

### Example 5 Expression, refolding and purification of a high affinity TCR

Expression vectors for TCR α and β chains were transferred into the expression bacteria BL21 (DE3) by chemical transformation, respectively. The bacteria were grown in an LB medium and induced with a final concentration of 0.5 mM IPTG at OD₆₀₀ = 0.6. The inclusion bodies formed after the TCR α and β chains were expressed were extracted by BugBuster Mix (Novagene) and repeatedly washed with a BugBuster solution. The inclusion bodies were finally dissolved in 6 M guanidine hydrochloride, 10 mM dithiothreitol (DTT), 10 mM ethylenediaminetetraacetic acid (EDTA), 20 mM Tris (pH 8.1).

The dissolved TCR α and β chains were rapidly mixed in 5 M urea, 0.4 M arginine, 20 mM Tris (pH 8.1), 3.7 mM cystamine, 6.6 mM β-mercapoethylamine (4 °C) at a mass ratio of 1:1. The final concentration was 60 mg/mL. After mixing, the solution was dialyzed against 10 volumes of deionized water (4 °C). After 12 h, deionized water was replaced with a buffer (20 mM Tris, pH 8.0) and dialysis was continued at 4 °C for 12 h. After completion of the dialysis, the solution was filtered through a 0.45 µM filter and purified through an anion-exchange column (HiTrap Q HP, 5 mL, GE Healthcare). The elution peak of the TCR containing the successfully refolded αβ dimer was confirmed by SDS-PAGE gel. The TCR was then further purified by gel permeation chromatography (HiPrep 16/60, Sephacryl S-100 HR, GE Healthcare). The purity of the purified TCR was determined by SDS-PAGE to be greater than 90%, and the concentration thereof was determined by the BCA method.

### Example 6 BIAcore analysis results

The affinity of the αβ heterodimeric TCR with high affinity CDRs introduced for KWVESIFLIF-HLA-A2402 complex was detected by the method described in Example 3.

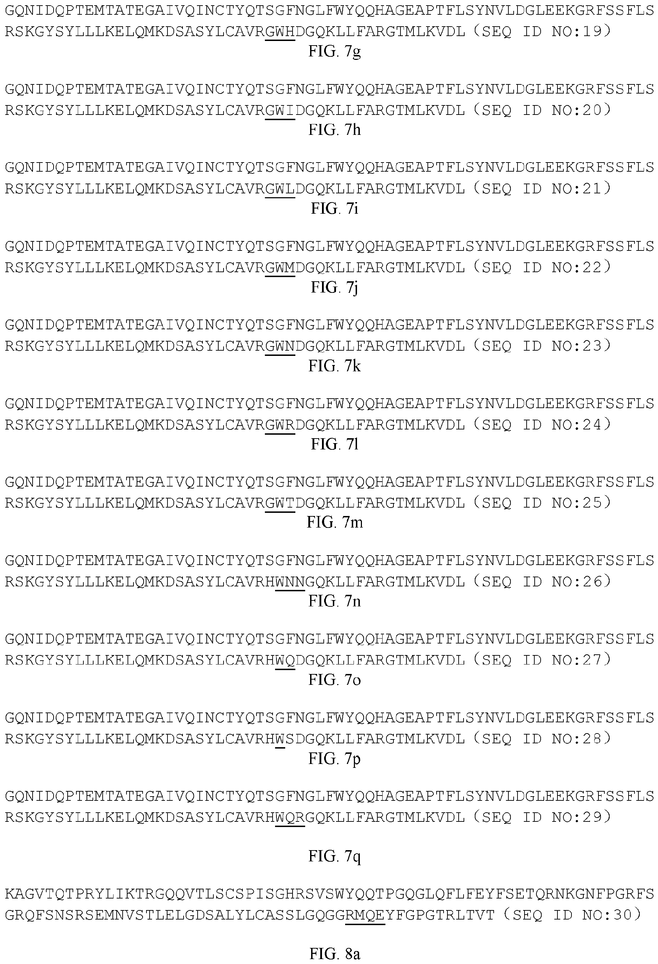

The amino acid sequences of α and β chain variable domains of the high affinity TCR obtained in the present disclosure are shown in FIGS. 7-1 to 7-17 and FIGS. 8-1 to 8-4, respectively. Since the CDRs of a TCR molecule determine the affinity of the TCR molecule for the corresponding pMHC complex, those skilled in the art can anticipate that the αβ heterodimeric TCR with high affinity mutation sites introduced also has high affinity for KWVESIFLIF-HLA-A2402 complex. An expression vector was constructed by the method described in Example 4, and the preceding αβ heterodimeric TCR with high affinity mutations introduced was expressed, refolded and purified by the method described in Example 5. The affinity of the TCR for KWVESIFLIF-HLA-A2402 complex was determined by BIAcore T200, as shown in Table 2.

**Table 2**

| TCR No. | Sequence of α chain variable domain SEQ ID NO: | Sequence of β chain variable domain SEQ ID NO: | K_{D} |
|---|---|---|---|
| 1 | 13 | 2 | 5.90E-07 |
| 2 | 14 | 2 | 7.21E-07 |
| 3 | 1 | 30 | 1.77E-06 |
| 4 | 15 | 2 | 5.56E-07 |
| 5 | 16 | 2 | 1.09E-06 |
| 6 | 1 | 31 | 1.53E-06 |
| 7 | 17 | 2 | 6.18E-06 |
| 8 | 1 | 32 | 1.11E-05 |
| 9 | 18 | 2 | 1.03E-06 |
| 10 | 19 | 2 | 5.14E-07 |
| 11 | 20 | 2 | 4.75E-06 |
| 12 | 21 | 2 | 1.08E-06 |
| 13 | 22 | 2 | 6.97E-07 |
| 14 | 23 | 2 | 3.80E-07 |
| 15 | 24 | 2 | 4.31E-05 |
| 16 | 25 | 2 | 2.55E-06 |
| 17 | 26 | 2 | 2.44E-06 |
| 18 | 27 | 2 | 4.88E-06 |
| 19 | 28 | 2 | 5.21E-06 |
| 20 | 1 | 33 | 3.01E-05 |
| 21 | 29 | 2 | 7.15E-06 |

As can be seen from Table 2, the affinity of the heterodimeric TCR for KWVESIFLIF-HLA-A2402 complex is at least twice that of the wild type TCR.

### Example 7 Expression, refolding and purification of fusions of anti-CD3 antibodies with αβ heterodimeric TCRs with high affinity

An anti-CD3 single-chain antibody (scFv) was fused with an αβ heterodimeric TCR to prepare a fusion molecule. The anti-CD3 scFv was fused with the β chain of the TCR, where the β chain of the TCR may comprise the β chain variable domain of any one of the above αβ heterodimeric TCRs with high affinity, and the TCR α chain of the fusion molecule may comprise the α chain variable domain of any one of the above αβ heterodimeric TCRs with high affinity.

Construction of an expression vector for the fusion molecule
1. Construction of an expression vector for the α chain: The target gene carrying the α chain of the αβ heterodimeric TCR was digested with NcoI and NotI and ligated with pET28a vector digested with NcoI and NotI. The ligation product was transferred into *E. coli* DH5α, plated on a kanamycin-containing LB plate, inverted and cultured at 37 °C overnight, and the positive clones were picked for PCR screening. Positive recombinants were sequenced to determine the correct sequence and the recombinant plasmid was extracted and transferred into *E. coli* Tuner (DE3) for expression.
2. Construction of an expression vector for anti-CD3 (scFv)-β chain: Primers were designed by overlap PCR to ligate the genes of the anti-CD3 scFv and the β chain of the heterodimeric TCR with high affinity. An intermediate linker was GGGGS (SEQ ID NO: 31), and the gene fragment of the fusion protein of the anti-CD3 scFv and the β chain of the heterodimeric TCR with high affinity had restriction enzyme sites NcoI (CCATGG (SEQ ID NO: 32)) and NotI (GCGGCCGC (SEQ ID NO: 33)). The PCR amplification product was digested with NcoI and NotI and ligated with pET28a vector digested with NcoI and NotI. The ligation product was transferred into *E*. *coli* DH5α competent cells, plated on a kanamycin-containing LB plate, inverted and cultured at 37 °C overnight, and the positive clones were picked for PCR screening. Positive recombinants were sequenced to determine the correct sequence and the recombinant plasmid was extracted and transferred into *E. coli* Tuner (DE3) competent cells for expression.

### Expression, refolding and purification of fusion proteins

The expression plasmids were separately transferred into *E. coli* Tuner (DE3) competent cells, plated on an LB plate (kanamycin, 50 µg/mL) and cultured overnight at 37 °C. On the next day, the clones were picked and inoculated into 10 mL of LB liquid medium (kanamycin, 50 µg/mL), cultured for 2-3 h, then inoculated to 1 L of LB medium at a volume ratio of 1:100, cultured until OD₆₀₀ was 0.5-0.8, and then induced to express proteins of interest using IPTG with a final concentration of 1 mM. Four hours after induction, the cells were harvested by centrifugation at 6000 rpm for 10 min. The bacteria cells were washed once in a PBS buffer and dispensed. Bacteria cells corresponding to 200 mL of the bacterial culture were taken and lysed with 5 mL of BugBuster Master Mix (Merck). The inclusion bodies were collected by centrifugation at 6000 g for 15 min. The inclusion bodies were washed 4 times with a detergent to remove cell debris and membrane components. The inclusion bodies were then washed with a buffer such as PBS to remove the detergent and salt. Finally, the inclusion bodies were dissolved in a buffer solution of 6M guanidine hydrochloride, 10 mM dithiothreitol (DTT), 10 mM ethylenediaminetetraacetic acid (EDTA), 20 mM Tris, pH 8.1, and the concentration of the inclusion bodies was determined. The inclusion bodies were dispensed and cryopreserved at -80 °C.

The dissolved TCR α chain and anti-CD3 (scFv)-β chain were rapidly mixed at a mass ratio of 2:5 in 5 M urea, 0.4 M L-arginine, 20 mM Tris pH 8.1, 3.7 mM cystamine and 6.6 mM β-mercapoethylamine (4 °C), and the final concentrations of the α chain and the anti-CD3 (scFv)-β chain were 0.1 mg/mL and 0.25 mg/mL, respectively.

After mixing, the solution was dialyzed against 10 volumes of deionized water (4°C). After 12 h, deionized water was replaced with a buffer (10 mM Tris, pH 8.0) and dialysis was continued at 4 °C for 12 h. After completion of the dialysis, the solution was filtered through a 0.45 µM filter and purified through an anion-exchange column (HiTrap Q HP, 5 mL, GE Healthcare). The elution peak of the TCR containing the successfully refolded TCR α chain and anti-CD3 (scFv)-β chain dimer was confirmed by SDS-PAGE gel. The TCR fusion molecule was then purified by size-exclusion chromatography (S-100 16/60, GE Healthcare) and further purified by an anion-exchange column (HiTrap Q HP, 5 mL, GE healthcare). The purity of the purified TCR fusion molecule was determined by SDS-PAGE to be greater than 90%, and the concentration thereof was determined by the BCA method.

### Example 8 Activation function assay of effector cells transfected with the high affinity TCR of the present disclosure for T2 cells loaded with short peptides

The function and specificity of the high affinity TCR of the present disclosure in cells are detected by an ELISPOT assay well-known to those skilled in the art. CD3+ T cells isolated from the blood of healthy volunteers were transfected with the high affinity TCR of the present disclosure as effector cells, and CD3+ T cells from the same volunteers were transfected with the wild type TCR (WT-TCR) and another TCR (A6) as control groups. The target cells used were T2-A24 (T2 cells transfected with HLA-A2402, the same below) loaded with AFP antigen short peptides KWVESIFLIF, and T2-A24 cells loaded with other short peptides and T2-A24 cells loaded with no short peptides were used as control groups. The high affinity TCRs are all the TCRs shown in Table 2, i.e., TCR1 to TCR21.

Firstly, an ELISPOT plate was prepared. The ELISPOT plate was activated and coated with ethanol overnight at 4 °C. On the first day of the assay, the coating solution was removed, the plate was washed, blocked and incubated at room temperature for 2 h, and the blocking solution was removed. Components of the assay were added to the ELISPOT plate: 1 × 10⁴ target cells/well, 2×10³ effector cells/well (calculated according to the positive rate of transfection), and two duplicate wells were set. The corresponding short peptides were added so that the final concentration of the short peptides in the wells of the ELISPOT plate was 1×10⁻⁶ M. The ELISPOT plate was incubated overnight (37 °C, 5% CO₂). On the second day of the assay, the plate was washed, subjected to secondary detection and development and dried, and the spots formed on the film were counted using an immunospot plate reader (ELISPOT READER system; AID20 Company).

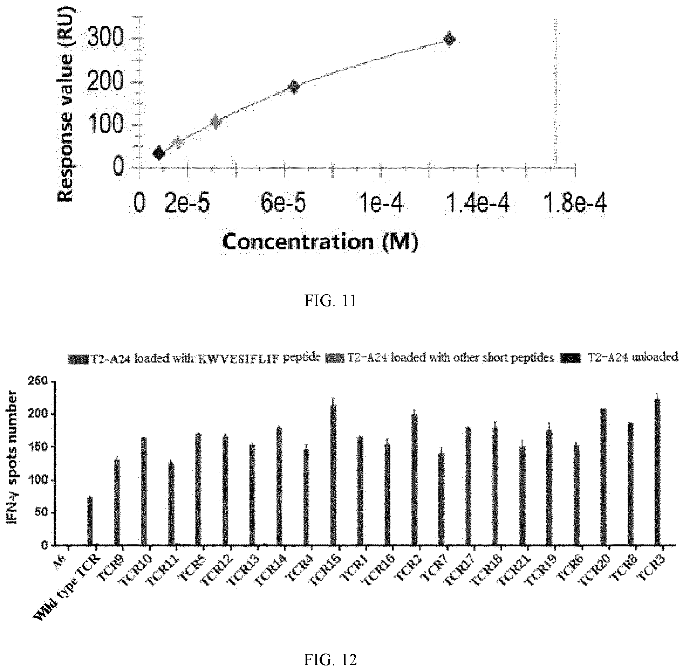

The results of the assay are shown in FIG. 12. For the target cells loaded with AFP antigen short peptides KWVESIFLIF, the T cells transfected with the high affinity TCR of the present disclosure exhibit a more obvious activation response that the T cells transfected with the wild type TCR, while the T cells transfected with another TCR have no activation state. Meanwhile, the T cells transfected with the high affinity TCR of the present disclosure are not activated by the target cells loaded with other short peptides or unloaded.

### Example 9 Activation function assay of effector cells transfected with the high affinity TCR of the present disclosure for a tumor cell line

This example also demonstrates that effector cells transfected with the high affinity TCR of the present disclosure have a good specific activation effect on target cells. The function and specificity of the high affinity TCR of the present disclosure in cells are detected by an ELISPOT assay well-known to those skilled in the art. CD3+ T cells isolated from the blood of healthy volunteers were transfected with the high affinity TCR of the present disclosure as effector cells, and CD3+ T cells from the same volunteers were transfected with another TCR (A6) as a control group. The high affinity TCRs are all the TCRs shown in Table 2, i.e., TCR1 to TCR21. Among the tumor cell lines used in the assay, HepG2 and SK-HEP-1-AFP (AFP overexpression) are positive tumor cell lines, while SK-HEP-1, Huh-1, MKN7 and NCI-H226 cells are negative tumor cell lines as control.

Firstly, an ELISPOT plate was prepared. The ELISPOT plate was activated and coated with ethanol overnight at 4 °C. On the first day of the assay, the coating solution was removed, the plate was washed, blocked and incubated at room temperature for 2 h, and the blocking solution was removed. Components of the assay were added to the ELISPOT plate in sequence: 2×10⁴ target cells/well, 2×10³ effector cells/well (calculated according to the positive rate of transfection), and two duplicate wells were set. The ELISPOT plate was incubated overnight (37 °C, 5% CO₂). On the second day of the assay, the plate was washed, subjected to secondary detection and development and dried, and the spots formed on the film were counted using an immunospot plate reader (ELISPOT READER system; AID20 Company).

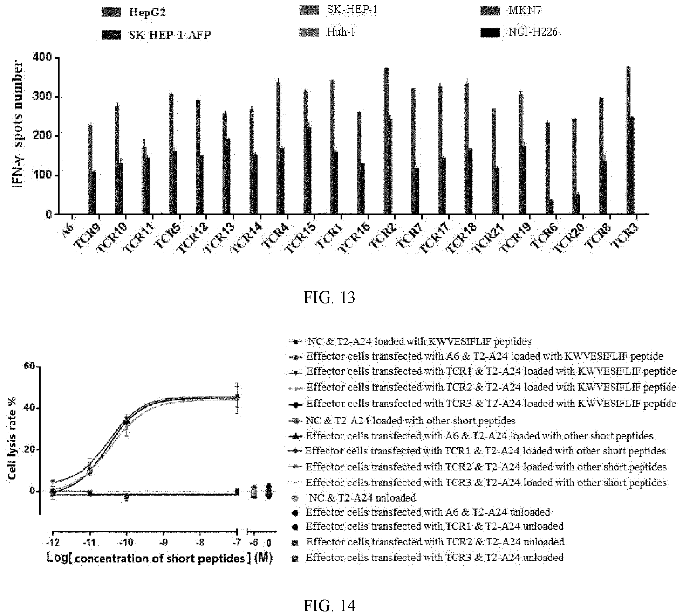

The results of the assay are shown in FIG. 13. The effector cells transfected with the high affinity TCR of the present disclosure can be well activated by the positive tumor cell lines, while the effector cells transfected with another TCR have no response. Meanwhile, the effector cells transfected with the high affinity TCR of the present disclosure have no activation effect on the negative tumor cell lines, which further demonstrates the antigenic specificity of the high affinity TCR.

### Example 10 Killing function assay of effector cells transfected with the high affinity TCR of the present disclosure for T2 cells loaded with gradients of short peptides

This example demonstrates the killing function of the cells transfected with the TCR of the present disclosure by measuring the release of LDH through a non-radioactive cytotoxicity assay well-known to those skilled in the art. The assay is a colorimetric alternative to the 51Cr release cytotoxicity assay and quantifies lactate dehydrogenase (LDH) released after cell lysis. The LDH released in a culture medium is detected using a 30-minute coupled enzymatic reaction where the LDH converts a tetrazolium salt (INT) to red formazan. The amount of the red product produced is proportional to the number of cells lysed. Visible absorbance data at 490 nm can be collected using a standard 96-well plate reader. Calculation formula: cytotoxicity (%) = 100% × (assay - spontaneous release of effector cells - spontaneous release of target cells)/(maximum release of target cells - spontaneous release of target cells)

In the LDH assay in this example, CD3+ T cells isolated from the blood of healthy volunteers were transfected with the high affinity TCR of the present disclosure as effector cells, and CD3+ T cells from the same volunteers were transfected with other TCR (A6) and empty transfected (NC) as control groups. The high affinity TCRs and their numbers are known from Table 2, which are TCR1 (α chain variable domain SEQ ID NO: 13 and β chain variable domain SEQ ID NO: 2), TCR2 (α chain variable domain SEQ ID NO: 14 and β chain variable domain SEQ ID NO: 2) and TCR3 (α chain variable domain SEQ ID NO: 1 and β chain variable domain SEQ ID NO: 30), respectively. The target cells were T2-A24 (T2 cells transfected with HLA-A2402, the same below) loaded with KWVESIFLIF peptides, and T2-A24 cells loaded with another antigen and T2-A24 cells loaded with no short peptides were used as control groups.

Firstly, an LDH plate was prepared. 3×10⁴ target cells/well and 3×10⁴ effector cells/well (calculated according to the positive rate of antibodies) were added to the corresponding wells, AFP antigen short peptides KWVESIFLIF were added to the experimental groups so that the final concentrations of the short peptides in the wells of the ELISPOT plate were at six gradients of 1×10⁻¹² M to 1×10⁻⁷ M, and other short peptides were added to the control group so that the final concentration of the short peptides was 1×10⁻⁶ M, where three duplicate wells were set. At the same time, effector cell spontaneous wells, target cell spontaneous wells, target cell maximum wells, volume correction control wells and medium background control wells were set. The LDH plate was incubated overnight (37 °C, 5% CO₂). On the second day of the assay, the cells were subjected to detection and development. After the reaction was terminated, the absorbance was recorded at 490 nm with a microplate reader (Bioteck).

The results of the assay are shown in FIG. 14. For the T2-A24 cells loaded with the gradients of AFP antigen short peptides KWVESIFLIF, the effector cells transfected with the high affinity TCR of the present disclosure have a strong killing effect and respond even at a low concentration of the above short peptides, while the effector cells transfected with another TCR have no killing effect all the way. Meanwhile, the effector cells transfected with the high affinity TCR of the present disclosure have no killing effect on the target cells loaded with other short peptides.

### Example 11 Killing function assay (LDH assay) of effector cells transfected with the high affinity TCR of the present disclosure for a tumor cell line

This example also demonstrates the killing function of the cells transfected with the TCR of the present disclosure by measuring the release of LDH through a non-radioactive cytotoxicity assay well-known to those skilled in the art.

In the LDH assay in this example, CD3+ T cells isolated from the blood of healthy volunteers were transfected with the high affinity TCR of the present disclosure as effector cells, and CD3+ T cells from the same volunteers were transfected with the wild type TCR (WT-TCR) and another TCR (A6) as control groups.

The assay was performed in two batches (I) and (II) of TCRs.
(I) The high affinity TCRs and their numbers are known from Table 2, which are TCR7 (α chain variable domain SEQ ID NO: 17 and β chain variable domain SEQ ID NO: 2), TCR17 (α chain variable domain SEQ ID NO: 26 and β chain variable domain SEQ ID NO: 2), TCR19 (α chain variable domain SEQ ID NO: 28 and β chain variable domain SEQ ID NO: 2) and TCR3 (α chain variable domain SEQ ID NO: 1 and β chain variable domain SEQ ID NO: 30), respectively. The positive tumor cell lines used in this batch were HepG2 and SK-HEP-1-AFP (AFP overexpression), while the negative tumor cell lines MKN1, NCI-H226 and SK-HEP-1 were used as control.
(II) The high affinity TCRs and their numbers are known from Table 2, which are TCR9 (α chain variable domain SEQ ID NO: 18 and β chain variable domain SEQ ID NO: 2), TCR10 (α chain variable domain SEQ ID NO: 19 and β chain variable domain SEQ ID NO: 2), TCR5 (α chain variable domain SEQ ID NO: 16 and β chain variable domain SEQ ID NO: 2), TCR14 (α chain variable domain SEQ ID NO: 23 and β chain variable domain SEQ ID NO: 2), TCR4 (α chain variable domain SEQ ID NO: 15 and β chain variable domain SEQ ID NO: 2), TCR6 (α chain variable domain SEQ ID NO: 1 and β chain variable domain SEQ ID NO: 31), TCR1 (α chain variable domain SEQ ID NO: 13 and β chain variable domain SEQ ID NO: 2) and TCR16 (α chain variable domain SEQ ID NO: 25 and β chain variable domain SEQ ID NO: 2), respectively. The positive tumor cell line used in this batch was HepG2, while the negative tumor cell lines NCI-H226 and SK-HEP-1 were used as control.

Firstly, an LDH plate was prepared. Components of the assay were added to the plate in sequence: 3×10⁴ target cells/well and 3×10⁴ effector cells/well were added to the corresponding wells, and three duplicate wells were set. At the same time, effector cell spontaneous wells, target cell spontaneous wells, target cell maximum wells, volume correction control wells and medium background control wells were set. The LDH plate was incubated overnight (37 °C, 5% CO₂). On the second day of the assay, the cells were subjected to detection and development. After the reaction was terminated, the absorbance was recorded at 490 nm with a microplate reader (Bioteck).

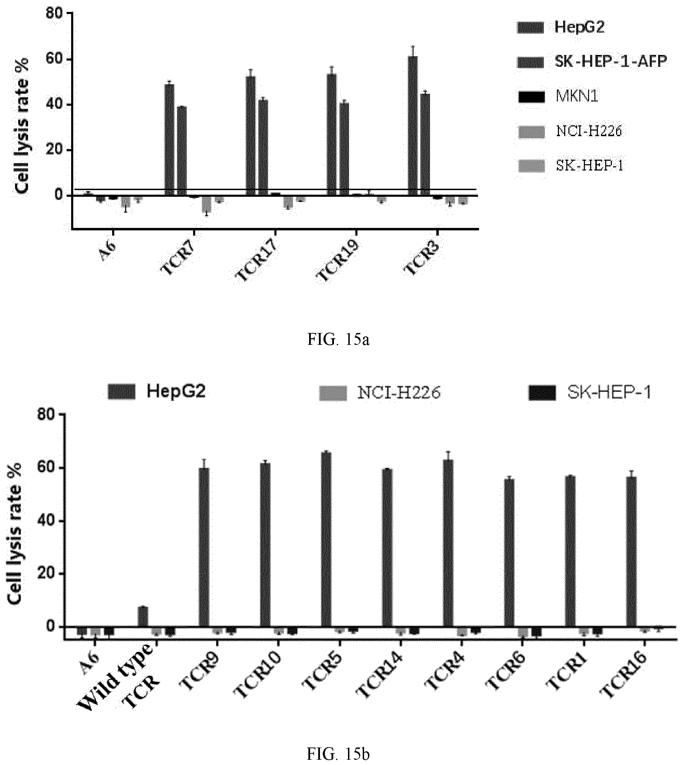

The results of the assay are shown in FIGS. 15a and 15b. Compared with the cells transfected with the wild type TCR, the cells transfected with the high affinity TCR of the present disclosure have a significantly enhanced killing effect on the positive tumor cell lines, while the effector cells transfected with another TCR almost have no killing effect. Meanwhile, the cells transfected with the high affinity TCR of the present disclosure almost have no killing effect on negative tumor cell lines.

### Example 12 Killing function verification (IncuCyte assay) of effector cells transfected with the high affinity TCR molecules of the present disclosure for a tumor cell line

This example further demonstrates that the effector cells transfected with the high affinity TCR of the present disclosure have a good specific killing effect on target cells through an IncuCyte assay well-known to those skilled in the art. IncuCyte is a functional analysis system that can automatically analyze images at different time points and quantify real-time cell apoptosis through real-time microscopic photography in an incubator.

CD3+ T cells isolated from the blood of healthy volunteers were transfected with the randomly selected TCRs of the present disclosure as effector cells, and CD3+ T cells from the same volunteers were transfected with another TCR (A6) and no TCR (NC) as control groups. The TCRs and their numbers are known from Table 2, which are TCR1 (α chain variable domain SEQ ID NO: 13 and β chain variable domain SEQ ID NO: 2), TCR2 (α chain variable domain SEQ ID NO: 14 and β chain variable domain SEQ ID NO: 2) and TCR3 (α chain variable domain SEQ ID NO: 1 and β chain variable domain SEQ ID NO: 30), respectively. Among the target cell lines, HepG2 is a positive tumor cell line and MKN7 is a negative tumor cell line as control.

On the first day of the assay, the target cells were digested, centrifuged, resuspended in a complete medium of RPMI1640+10%FBS without phenol red, and evenly spread on a 96-well plate: 2×10⁴ target cells/well. The plate was put back in a 37 °C, 5% CO₂ incubator and incubated overnight. On the second day, the medium in the 96-well plate was discarded and replaced with a phenol red-free RPMI1640+10%FBS medium containing dye caspase3/7 reagent, where the concentration of the dye was 2 drops/mL. The old medium was discarded and replaced with a new phenol red-free RPMI1640+10%FBS medium. 1×10⁴ effector cells/well (calculated according to the positive rate of transfection) were incubated with an experimental group plated with target cells. The plate was incubated for half an hour in a real-time dynamic live cell imaging analyzer IncuCyte ZooM dedicated for IncuCyte detection and observed and photographed in real time. The detection results were processed and the data was analyzed and exported using IncuCyte ZooM 2016A.

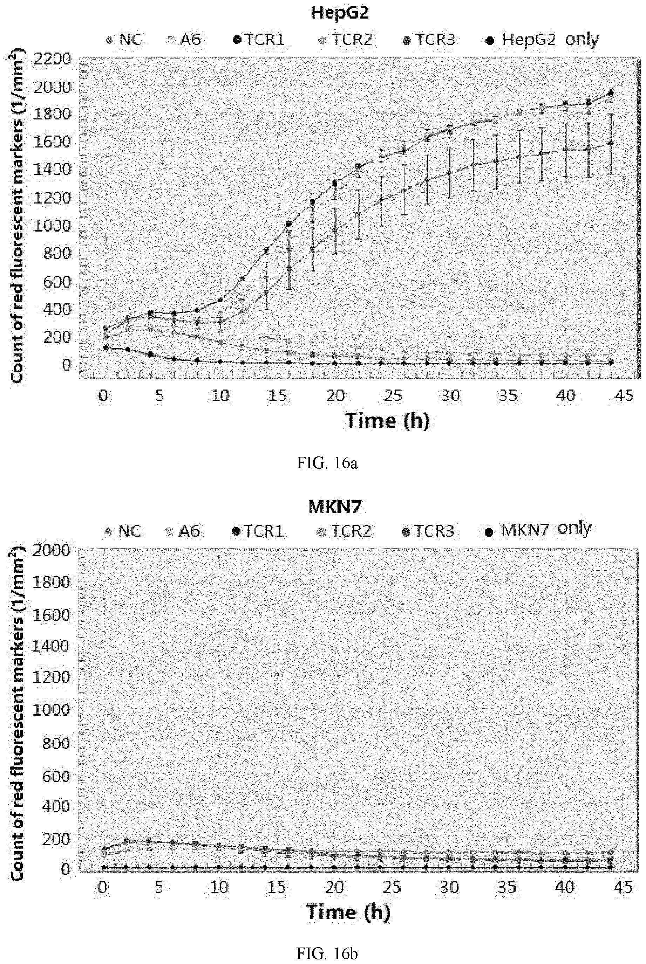

As shown by the detection results in FIGS. 16a and 16b, the cells transfected with the high affinity TCR of the present disclosure can exhibit a very strong killing effect on the positive tumor cell line in a short period of time, while the effector cells transfected with another TCR almost have no killing effect. Meanwhile, the cells transfected with the high affinity TCR of the present disclosure almost have no killing effect on the negative tumor cells expressing no relevant antigens.

All documents mentioned in the present disclosure are hereby incorporated by reference in their entireties as if each is incorporated by reference. In addition, it is to be understood that after reading the teachings of the present disclosure, those skilled in the art can make various changes or modifications on the present disclosure, and these equivalent forms also fall within the scope as defined by the appended claims of the present application.

## Claims

1. A T-cell receptor (TCR), wherein the TCR comprises a TCRα chain variable domain and a TCRβ chain variable domain and has an activity of binding to KWVESIFLIF-HLA A2402 complex, and the TCRα chain variable domain comprises an amino acid sequence having at least 90% of sequence homology with an amino acid sequence as shown in SEQ ID NO: 1, and the TCRβ chain variable domain comprises an amino acid sequence having at least 90% of sequence homology with an amino acid sequence as shown in SEQ ID NO: 2.

2. The TCR of claim 1, wherein the affinity of the TCR for KWVESIFLIF-HLA A2402 complex is at least twice that of a wild type TCR.

3. The TCR of claim 1, wherein the TCRα chain variable domain comprises an amino acid sequence having at least 95% of sequence homology with the amino acid sequence as shown in SEQ ID NO: 1, and/or the TCRβ chain variable domain comprises an amino acid sequence having at least 95% of sequence homology with the amino acid sequence as shown in SEQ ID NO: 2.

4. The TCR of claim 1, wherein three complementarity-determining regions (CDRs) of the TCRβ chain variable domain are:
CDR1β: SGHRS;
CDR2β: YFSETQ; and
CDR3β: ASSLGQGGKDEQY;
preferably, the TCRβ chain variable domain comprises the amino acid sequence as shown in SEQ ID NO: 2.

5. The TCR of claim 1, wherein the TCRα chain variable domain comprises an amino acid sequence having at least 97% of sequence homology with the amino acid sequence as shown in SEQ ID NO: 1, and three CDRs of the TCRβ chain variable domain are:
CDR1β: SGHRS;
CDR2β: YFSETQ; and
CDR3β: ASSLGQGGKDEQY;
preferably, the TCRα chain variable domain comprises an amino acid sequence having at least 97% of sequence homology with the amino acid sequence as shown in SEQ ID NO: 1, and the TCRβ chain variable domain comprises the amino acid sequence as shown in SEQ ID NO: 2.

6. The TCR of claim 1, wherein the TCRα chain variable domain comprises an amino acid sequence having at least 97% of sequence homology with the amino acid sequence as shown in SEQ ID NO: 1, and/or the TCRβ chain variable domain comprises an amino acid sequence having at least 96% of sequence homology with the amino acid sequence as shown in SEQ ID NO: 2.

7. The TCR of claim 1, wherein three CDRs of the TCRα chain variable domain have the following reference sequences:
CDR1α: TSGFNG
CDR2α: NVLDGL
CDR3α: AVRHFSDGQKLL, and three CDRs of the TCRβ chain variable domain have the following reference sequences:
CDR1β: SGHRS
CDR2β: YFSETQ
CDR3β: ASSLGQGGKDEQY;
and the TCR contains at least one of the following mutations in CDRs of an α chain of the TCR:
| Residue Before Mutation | Residue After Mutation |
|---|---|
| H at position 4 of CDR3α | G |
| F at position 5 of CDR3α | W |
| G at position 6 of CDR3α | Y or Q or K or D or A or H or I or L or M or N or R or T |
| D at position 7 of CDR3α | R or G or N |
;
and/or the TCR contains at least one of the following mutations in CDRs of a β chain of the TCR:
| Residue Before Mutation | Residue After Mutation |
|---|---|
| S at position 3 of CDR3β | A |
| L at position 4 of CDR3β | S |
| Q at position 6 of CDR3β | V |
| K at position 9 of CDR3β | R |
| D at position 10 of CDR3β | M or G |
| E at position 11 of CDR3β | Q or T |
| Q at position 12 of CDR3β | E or L |

8. The TCR of claim 7, wherein CDR3α contains the following amino acid mutation:
| Residue Before Mutation | Residue After Mutation |
|---|---|
| H at position 4 of CDR3α | G |
; and/or CDR3α contains the following amino acid mutation:
| Residue Before Mutation | Residue After Mutation |
|---|---|
| F at position 5 of CDR3α | W |

9. The TCR of claim 7, wherein CDR3α contains the following amino acid mutations:
| Residue Before Mutation | Residue After Mutation |
|---|---|
| H at position 4 of CDR3α | G |
| F at position 5 of CDR3α | W |

10. The TCR of claim 7, wherein CDR3α of the TCR is selected from AVRHFSDGQKLL, AVRGWSDGQKLL, AVRGWYDGQKLL and AVRGWQDGQKLL.

11. The TCR of claim 10, wherein CDR3β of the TCR is selected from ASSLGQGGKDEQY and ASSLGQGGRMQEY.

12. The TCR of claim 1, wherein the TCR has CDRs selected from the group consisting of:
| CDR No. | α-CDR1 | α-CDR2 | α-CDR3 | β-CDR1 | β-CDR2 | β-CDR3 |
|---|---|---|---|---|---|---|
| 1 | TSGFN G | NVLD GL | AVRGWSDGQK LL | SGHR S | YFSET Q | ASSLGQGGKDE QY |
| 2 | TSGFN G | NVLD GL | AVRGWYDGQK LL | SGHR S | YFSET Q | ASSLGQGGKDE QY |
| 3 | TSGFN G | NVLD GL | AVRHFSDGQKL L | SGHR S | YFSET Q | ASSLGQGGRM QEY |
| 4 | TSGFN | NVLD | AVRGWQDGQK | SGHR | YFSET | ASSLGQGGKDE |
| | G | GL | LL | S | Q | QY |
| 5 | TSGFN G | NVLD GL | AVRGWKDGQK LL | SGHR S | YFSET Q | ASSLGQGGKDE QY |
| 6 | TSGFN G | NVLD GL | AVRHFSDGQKL L | SGHR S | YFSET Q | ASASGVGGKDE QY |
| 7 | TSGFN G | NVLD GL | AVRHWDGGQK LL | SGHR S | YFSET Q | ASSLGQGGKDE QY |
| 8 | TSGFN G | NVLD GL | AVRHFSDGQKL L | SGHR S | YFSET Q | ASSLGQGGRGT LY |
| 9 | TSGFN G | NVLD GL | AVRGWADGQK LL | SGHR S | YFSET Q | ASSLGQGGKDE QY |
| 10 | TSGFN G | NVLD GL | AVRGWHDGQK LL | SGHR S | YFSET Q | ASSLGQGGKDE QY |
| 11 | TSGFN G | NVLD GL | AVRGWIDGQK LL | SGHR S | YFSET Q | ASSLGQGGKDE QY |
| 12 | TSGFN G | NVLD GL | AVRGWLDGQK LL | SGHR S | YFSET Q | ASSLGQGGKDE QY |
| 13 | TSGFN G | NVLD GL | AVRGWMDGQ KLL | SGHR S | YFSET Q | ASSLGQGGKDE QY |
| 14 | TSGFN G | NVLD GL | AVRGWNDGQK LL | SGHR S | YFSET Q | ASSLGQGGKDE QY |
| 15 | TSGFN G | NVLD GL | AVRGWRDGQK LL | SGHR S | YFSET Q | ASSLGQGGKDE QY |
| 16 | TSGFN G | NVLD GL | AVRGWTDGQK LL | SGHR S | YFSET Q | ASSLGQGGKDE QY |
| 17 | TSGFN G | NVLD GL | AVRHWNNGQK LL | SGHR S | YFSET Q | ASSLGQGGKDE QY |
| 18 | TSGFN G | NVLD GL | AVRHWQDGQK LL | SGHR S | YFSET Q | ASSLGQGGKDE QY |
| 19 | TSGFN G | NVLD GL | AVRHWSDGQK LL | SGHR S | YFSET Q | ASSLGQGGKDE QY |
| 20 | TSGFN G | NVLD GL | AVRHFSDGQKL L | SGHR S | YFSET Q | ASSLGQGGRDI QY |
| 21 | TSGFN G | NVLD GL | AVRHWQRGQK LL | SGHR S | YFSET Q | ASSLGQGGKDE QY |

13. The TCR of claim 1, wherein the TCR is soluble.

14. The TCR of claim 1, wherein the TCR is an αβ heterodimeric TCR and comprises an α chain TRAC constant region sequence and a β chain TRBC1 or TRBC2 constant region sequence.

15. The TCR of claim 1, wherein the TCR comprises (i) a TCRα chain variable domain and all or part of a TCR α chain constant region other than a transmembrane domain and (ii) a TCRβ chain variable domain and all or part of a TCR β chain constant domain other than a transmembrane domain.

16. The TCR of claim 1, wherein an artificial interchain disulfide bond is contained between an α chain constant region and a β chain constant region of the TCR;
preferably, one or more groups of amino acids selected from the following are substituted by cysteine residues forming the artificial interchain disulfide bond between the α chain constant region and the β chain constant region of the TCR:
Thr48 ofTRAC*01 exon 1 and Ser57 of TRBC1^{∗}01 or TRBC2*01 exon 1;
Thr45 ofTRAC*01 exon 1 and Ser77 of TRBC1^{∗}01 or TRBC2*01 exon 1;
Tyr10 ofTRAC*01 exon 1 and Ser17 of TRBC1^{∗}01 or TRBC2*01 exon 1;
Thr45 ofTRAC*01 exon 1 and Asp59 of TRBC1 *01 or TRBC2*01 exon 1;
Ser15 ofTRAC*01 exon 1 and Glu15 of TRBC1^{∗}01 or TRBC2*01 exon 1;
Arg53 ofTRAC*01 exon 1 and Ser54 of TRBC1^{∗}01 or TRBC2*01 exon 1;
Pro89 ofTRAC*01 exon 1 and Ala19 of TRBC1^{∗}01 or TRBC2*01 exon 1; and
Tyr10 ofTRAC*01 exon 1 and Glu20 of TRBC1^{∗}01 or TRBC2*01 exon 1.

17. The TCR of claim 1, wherein the TCRα chain variable domain comprises an amino acid sequence as shown in one of SEQ ID NOs: 1 and 13-29; and/or the TCRβ chain variable domain comprises an amino acid sequence as shown in one of SEQ ID NOs: 2 and 30-33;
preferably, the TCR is selected from the group consisting of:
| TCR No. | Sequence of α chain variable domain | Sequence of β chain variable domain |
|---|---|---|
| | | |
| | SEQ ID NO: | SEQ ID NO: |
|---|---|---|
| 1 | 13 | 2 |
| 2 | 14 | 2 |
| 3 | 1 | 30 |
| 4 | 15 | 2 |
| 5 | 16 | 2 |
| 6 | 1 | 31 |
| 7 | 17 | 2 |
| 8 | 1 | 32 |
| 9 | 18 | 2 |
| 10 | 19 | 2 |
| 11 | 20 | 2 |
| 12 | 21 | 2 |
| 13 | 22 | 2 |
| 14 | 23 | 2 |
| 15 | 24 | 2 |
| 16 | 25 | 2 |
| 17 | 26 | 2 |
| 18 | 27 | 2 |
| 19 | 28 | 2 |
| 20 | 1 | 33 |
| 21 | 29 | 2 |

18. The TCR of claim 1, wherein the TCR is a single chain TCR; preferably, the TCR is a single chain TCR consisting of an α chain variable domain and a β chain variable domain, wherein the α chain variable domain and the β chain variable domain are linked by a flexible short peptide sequence (linker).

19. The TCR of claim 1, wherein a conjugate binds to an α chain and/or a β chain of the TCR at C- or N-terminal; preferably, the conjugate is a detectable label or a therapeutic agent; more preferably, the therapeutic agent is an anti-CD3 antibody.

20. A multivalent TCR complex, comprising at least two TCR molecules, wherein at least one of the TCR molecules is the TCR of any one of the preceding claims.

21. A nucleic acid molecule, comprising a nucleic acid sequence for encoding the TCR of any one of claims 1 to 19 or a complementary sequence thereof.

22. A vector comprising the nucleic acid molecule of claim 21.

23. A host cell comprising the vector of claim 34 or having the exogenous nucleic acid molecule of claim 21 integrated into a chromosome of the host cell.

24. An isolated cell transduced with the TCR of any one of claims 1 to 19.

25. A pharmaceutical composition comprising a pharmaceutically acceptable carrier and the TCR of any one of claims 1 to 19 or the TCR complex of claim 20 or the cell of claim 24.

26. A method for treating a disease, comprising administering the TCR of any one of claims 1 to 19 or the TCR complex of claim 20 or the cell of claim 24 or the pharmaceutical composition of claim 25 to a subject in need thereof; preferably, the disease is AFP positive tumor; more preferably, the tumor is liver cancer.

27. A use of the T-cell receptor of any one of claims 1 to 19 or the TCR complex of claim 20 or the cell of claim 24 for preparation of a medicament for treating a tumor; preferably, the tumor is AFP positive tumor; more preferably, the tumor is liver cancer.

28. A method for preparing the T-cell receptor of any one of claims 1 to 19, comprising the steps of:
(i) culturing the host cell of claim 23 to express the T-cell receptor of any one of claims 1 to 19;
(ii) isolating or purifying the T-cell receptor.
